(19) Europäisches Patentamt / European Patent Office / Office européen des brevets

(11) **EP 1 832 607 B1**

(12) **EUROPEAN PATENT SPECIFICATION**

(45) Date of publication and mention
of the grant of the patent:
**01.07.2015 Bulletin 2015/27**

(51) Int Cl.:
*C08F 8/12* (2006.01)      *C08F 2/20* (2006.01)
*A61M 1/02* (2006.01)      *A61M 1/36* (2006.01)
*C08F 218/04* (2006.01)    *C08F 226/06* (2006.01)
*C08F 216/06* (2006.01)    *B01J 20/26* (2006.01)
*C08F 218/08* (2006.01)    *C08F 265/06* (2006.01)
*C08F 220/14* (2006.01)    *C08F 220/28* (2006.01)

(21) Application number: **05822683.8**

(22) Date of filing: **28.12.2005**

(86) International application number:
**PCT/JP2005/024091**

(87) International publication number:
**WO 2006/070876 (06.07.2006 Gazette 2006/27)**

(54) **CROSSLINKED POLYMER PARTICLE AND PROCESS FOR PRODUCING THE SAME**

VERNETZTES POLYMERTEILCHEN UND HERSTELLUNGSVERFAHREN DAFÜR

PARTICULES DE POLYMERE RETICULE ET PROCEDE SERVANT A PRODUIRE CELLES-CI

(84) Designated Contracting States:
**AT BE BG CH CY CZ DE DK EE ES FI FR GB GR HU IE IS IT LI LT LU LV MC NL PL PT RO SE SI SK TR**

(30) Priority: **28.12.2004   JP 2004380727**
**26.08.2005   JP 2005246090**
**08.11.2005   JP 2005323498**

(43) Date of publication of application:
**12.09.2007   Bulletin 2007/37**

(73) Proprietor: **Kaneka Corporation**
**Osaka (JP)**

(72) Inventors:
• **MORI, Toshiyuki,**
**KANEKA CORPORATION, Osaka Plant**
**Settsu-shi, Osaka 5660072 (JP)**
• **FUJITA, Koji,**
**KANEKA CORPORATION, Osaka Plant**
**Settsu-shi, Osaka 5660072 (JP)**
• **USHIZAKI, Koshin,**
**KANEKA CORPORATION, Osaka Plant**
**Settsu-shi, Osaka 5660072 (JP)**

(74) Representative: **Gille Hrabal**
**Brucknerstrasse 20**
**40593 Düsseldorf (DE)**

(56) References cited:
**EP-A2- 0 043 074      JP-A- 56 151 703**
**JP-A- 57 190 003      JP-A- 58 012 656**
**JP-A- 58 109 504      JP-A- 60 179 404**

• **DATABASE WPI Week 198526 Thomson Scientific, London, GB; AN 1985-157164 XP000002654714, & JP 60 090038 A (ASAHI CHEM IND CO LTD) 21 May 1985 (1985-05-21) & DATABASE CA [Online] CHEMICAL ABSTRACTS SERVICE, COLUMBUS, OHIO, US; 28 December 1985 (1985-12-28), "Blood-compatible adsorbents for removal of pathogenic substances from blood", retrieved from STN Database accession no. 103:220850 -& JP 60 090038 A (ASAHI CHEMICAL INDUSTRY CO., LTD., JAPAN) 21 May 1985 (1985-05-21)**

**Description**

TECHNICAL FIELD

The present invention relates to cross-linked polymer particle useful as processing material of liquid containing physiologically active substance and/or cells, etc., particularly body fluids such as blood and plasma, and as carrier thereof. Also, the invention relates to manufacturing method of polymer particle useful as adsorbent/processing material that processes body fluids and carrier thereof.

BACKGROUND ART

Extracorporeal therapy has recently prevailed as a treatment of various refractory diseases, for which satisfactory improvement has not been achieved by drug administration, etc. The extracorporeal therapy is a method in which patient's blood, etc. are led outside of the body to remove substances and cells, which exist in the blood, etc. and are closely associated with disease, and to reduce their levels, and after some treatment-related effects are provided, they are returned into the patient's body.

One example is blood purification by so-called plasma perfusion method, in which plasma separation membrane and plasma component are separated by centrifugation from the patient's blood that is led outside of the body, and after processing of said plasma with adsorbent, returned into the patient's body together with blood cell component; various adsorbers have been developed. In addition, so-called direct blood perfusion method, in which blood is directly processed without separating plasma component from patient's blood, is recently attracting attention.

Preferably the adsorbents used for them do not activate coagulation system and complement system. Marked activation of coagulation system may cause serious problems such as embolization in patient's body. In addition, activated complements C3a and C5a, generated in the process of the activation of complement system, have strong biological effects as anaphylatoxins and cause problems such as promotion of leukocyte chemotaxis, increased vascular permeability, decreased blood pressure, anaphylactic reaction, etc.

In addition, preferably, the adsorbents for direct processing of blood do not cause nonspecific adsorption and activation of blood cells. As is well known, blood cells brought out of the body are physiologically very unstable and are likely to cause adsorption and activation in contact with foreign matters such as adsorbent. Particularly, activation of leukocytes causes release of various physiologically active substances with resultant various problems.

Traditionally, natural polymers such as cellulose and agarose, synthetic polymers such as polyvinyl alcohol and polyhydroxy ethyl methacrylate, copolymer and polymer blend containing them and materials coated with them have been tried as these adsorbents and carriers thereof. However, information about the effects of the properties of these materials on the activation of coagulation and complement systems has been limited.

These adsorbents are often filled into a container with inlet and outlet, such as column, for use. In the case of particulate adsorbents, the effects of mean particle size on the properties of adsorbent have traditionally been known. For example, generally, smaller mean particle size has advantages in adsorption performance, but tends to reduce the passage of object liquid.

The particle size distribution of these adsorbents, particularly adsorbents made of synthetic polymers, has not fully been studied. This is due to the restriction on manufacturing, i.e., it's been difficult to obtain uniformly-sized polymer particles consisting of synthetic polymers suitable for the processing of body fluid, etc. For example, polymer particles of wide particle size distribution, containing large amounts of small and large particles, are produced by suspension polymerization method, a commonly-used manufacturing method of these polymer particles. The small and large particles can be reduced by conducting classification procedures such as screening, however, pursuit of more uniformly-sized particles causes significant classification loss and thus it is difficult to obtain uniformly-sized particles in a practical manner. For this reason, some degree of variation in particle size distribution had to be accepted, and reduced productivity due to classification loss was inevitable.

In Japanese Unexamined Patent Publication No. 58-12656, adsorbent for extracorporeal therapy that retains ligand on particulate rigid gel carrier with specific surface area of at least 5 $m^2$/g, adsorbent for extracorporeal therapy that is cross-linked synthetic polymer having hydroxyl group in said rigid gel carrier, whose mean particle size, water retention and hydroxyl density are within specified ranges, adsorbent for extracorporeal therapy that is cross-linked synthetic polymer in which said rigid gel carrier contains vinyl alcohol unit as a main component, adsorbent for extracorporeal therapy that is cross-linked synthetic polymer in which said rigid gel carrier can be obtained through hydrolysis of vinyl compound copolymers having carboxylic acid vinyl ester and isocyanurate ring are disclosed. Here, a method to obtain said absorbent by suspension polymerization were disclosed; loss of plasma proteins (albumin and complements), reduced blood cell count and the presence or absence of remaining blood and blood clot after passage of human plasma and human whole blood through said adsorbents were examined. However, no quantitative study was conducted on coagulation system and complement system or activation of leukocytes, and concerns about them were not resolved.

No study was conducted on the particle size distribution of said adsorbents, either.

Ichikawa, et al. [Journal of Artificial Organs 12 (1), pp 116 to 119 (1983)] showed an example of direct blood perfusion in mongrel adult dogs using adsorbent with tryptophan immobilized on porous gel of 74 to 210 μm diameter, consisting of polyvinyl alcohol gel, as ligand. However, no quantitative study was conducted on complement system and activation of leukocytes, and concerns about them were not resolved. The adsorbents used were those of wide particle size distribution, containing large amounts of small and large particles, as indicated by scanning microscopy, and neither study nor suggestion has been given on uniformly-sized adsorbent.

In Japanese Unexamined Patent Publication No. 63-115572, it was disclosed that even adsorbents of relatively small volume-weighted mean diameter, when having specific particle size distribution, allows blood perfusion without causing a marked increase of pressure loss and hemolysis. However, only natural polymer, cellulosic material, was exemplified, and no mention was made of synthetic polymer. No study was conducted to respond to the concerns about coagulation system and complement system or activation of leukocytes.

In Japanese Unexamined Patent Publication No. 2-199137, a manufacturing method of foamable thermoplastic polymer particles with uniform size distribution was disclosed. However, only a manufacturing method of foamable thermoplastic polymer particles, useful to achieve foam molding with excellent appearance and strength, was indicated; however, polymer particles, excellent in the passage of liquids containing physiologically active substances and/or cells, etc., such as body fluid, and less likely to activate coagulation system, complement system and cells, and a manufacturing method thereof were not indicated.

JP 60-090038 A discloses an insoluble carrier comprising a derivative of natural high-molecular cellulose having a hydroxyl group, a polymer or copolymer of a monomer having a hydroxyl group or a polymer or a copolymer comprising a monomer such as an aryl alcohol and its derivative and a polymer or copolymer having an ester group and forming a hydroxyl group by partial hydrolysis of said ester group.

EP 0 043 074 A discloses a granular crosslinked copolymer consisting of units of at least one vinyl alcohol, units of at least one vinyl ester of a carboxylic acid, and units of at least one crosslinking monomer having an isocyanurate ring.

JP 56-151703 A discloses a method for the preparation of a polyvinyl alcohol gel by copolymerizing vinyl acetate with a crosslinking agent having a triazine structure.

JP 60-1 79404 A discloses a crosslinked copolymer.

JP 57-190003 A discloses a porous activated gel which comprises a matrix of a totally porous crosslinked copolymer comprising as main components vinyl alcohol monomer units and crosslinkable monomer units and having a specific surface area of 5 to 1000 m$^2$/g and an reactive group linked to said matrix by a covalent bond.

JP 58-109504 A discloses a copolymer prepared by converting ester groups of a copolymer obtained from a vinyl carboxylate monomer and an isocyanurate ring containing crosslinking monomer into hydroxyl groups.

DISCLOSURE OF INVENTION

The object of the invention is to provide cross-linked polymer particles that hardly activate coagulation system, complement system and leukocytes, useful as an adsorbent that processes liquids containing physiologically active substances and/or cells, etc., such as body fluid, and carriers thereof. Additionally, the object is to provide cross-linked polymer particles that cause little loss of useful compounds due to nonspecific adsorption, etc. and little activation and adhesion of cells. Additionally, the object is to provide said cross-linked polymer particles that allow stable circulation of these highly viscous liquids at high speed and keep particle outflow at an extremely low level. Additionally, the object is to provide a manufacturing method of said polymer particles, which does not cause large amounts of classification loss.

As a result of a keen examination to solve the above mentioned traditional problems of adsorbent, the inventors surprisingly found that adsorbents, which activated coagulation system and complement system or leukocytes at an extremely low level, and thus could be used more safely, and carriers thereof could be developed using the cross-linked polymer particle in the invention.

The inventors also found that adsorbents and processing materials could be developed, in which said cross-linked polymer particles had appropriate degree of plasticity in aqueous medium, less susceptible to deformities by external force, such as pressure loss during liquid feeding, less likely to be broken and chipped off, and generated only small amounts of fine particles.

The inventors also found that extremely excellent passage of liquid containing highly viscous blood and plasma etc. and/or cells, higher factors of safety and excellent processing efficiency could be achieved by the use of said cross-linked polymer particles within specific ranges of mean particle size and particle size distribution.

The inventors also found an efficient manufacturing method of said polymer particles, once difficult to obtain, and achieved this invention.

Specifically, the present invention relates to cross-linked polymer particles comprising vinyl alcohol unit and nitrogen-containing polymerization unit as components, wherein nitrogen content In dry weight, based on the total weight of said particle, determined by element analysis, is 7.0 to 13.0 weight %, and surface nitrogen content against said particle surface, determined by X-ray photoelectron spectroscopy, is 5.0 to 15.0 at %, wherein said vinyl alcohol unit is formed through hydrolysis and/or ester exchange reaction of part or all of carboxylic vinyl ester units of cross-linked polymer that contains carboxylic vinyl ester unit obtained by polymerization of monomer mixture containing carboxylic vinyl ester and vinyl compound having triazine ring, and wherein the polymerization conversion rate of carboxylic vinyl ester in the polymerization of monomer mixture containing carboxylic vinyl ester and vinyl compound having triazine ring is 10 to 80 %.

The difference between the percentage of said nitrogen content and the percentage of said surface nitrogen content is preferably less than 2.0. The vinyl alcohol unit in said cross-linked polymer particle Is preferably formed through hydrolysis and/or ester exchange reaction of part or all of carboxylic vinyl ester units of cross-linked polymer that contains carboxylic vinyl ester unit obtained by polymerization of monomer mixture containing vinyl compound having carboxylic vinyl ester and vinyl compound having triazine ring.

The polymerization conversion rate of carboxylic vinyl ester in the polymerization of monomer mixture that contains vinyl compound having carboxylic vinyl ester and triazine ring is preferably 10 to 80 %.

Preferably, the volume-weighted mean diameter of said cross-linked polymer particles Is 50 to 3,000 $\mu$m, and at least 80 volume % of said particles have 0.8 to 1.2-fold volume-weighted mean diameter.

The particles of less than 100 $\mu$m particle size preferably account for 5 volume %.

The invention also relates to the processing material of body fluid, made of said cross-linked polymer particles.

Furthermore, the invention relates to a manufacturing method of polymer particles, including a process (a) to form uniformly-sized liquid drops of monomer mixture comprising vinyl alcohol unit and nitrogen-containing polymerization unit In dispersion medium and a process (b) to polymerize said liquid drops under the condition that neither connation nor additional dispersion occurs, wherein volume-weighted mean diameter is 50 to 3,000 $\mu$m, and 80 volume % of said particles have 0.8 to 1.2-fold volume-weighted mean diameter, wherein nitrogen content in dry weight against the total weight of said particle, determined by element analysis, is 7.0 to 13.0 weight %, and surface nitrogen content against said particle surface, determined by X-ray photoelectron spectrometry, is 5.0 to 15.0 at %, wherein said vinyl alcohol unit is formed through hydrolysis and/or ester exchange reaction of part or all of carboxylic vinyl ester units of cross-linked polymer that contains carboxylic vinyl ester unit obtained by polymerization of monomer mixture containing carboxylic vinyl ester and vinyl compound having triazine ring, and wherein the polymerization conversion rate of carboxylic vinyl ester in the polymerization of monomer mixture containing carboxylic vinyl ester and vinyl compound having triazine ring is 10 to 80 %.

In addition, a process (c) for processing and modification can be included after said process (b).

Preferably, the volume-weighted mean diameter of polymer particles Is 150 to 3,000 $\mu$m, and particles of less than 100 $\mu$m particle size account for at most 5 volume %. Polymer particles preferably have at least one kind of hydrophilic group, selected from hydroxyl, polyoxyethylene and pyrrolidone.

In addition, at least 10 weight % of all monomers preferably have pore structure obtained by the use of monomer mixture (i.e., cross-linking monomers).

Monomer mixture preferably contains at least one kind of monomer, selected from the group consisting of carboxylic vinyl ester, methacrylate ester, acrylic ester, aromatic vinyl compound and vinyl cyanide compound.

Vinyl alcohol unit is preferably obtained through a process to polymerize monomer mixture containing carboxylic vinyl ester and polyfunctional vinyl compound to form cross-linked polymer containing carboxylic vinyl ester unit as a compound, and a process to turn part or all of said carboxylic vinyl ester unit into vinyl alcohol units through hydrolysis and/ester exchange reaction.

Particle surface is preferably modified by polymerization of monomer mixture containing at least one kind of monomer selected from the group consisting of carboxylic vinyl ester, methacrylate ester, acrylic ester, aromatic vinyl compound and vinyl cyanide compound, and simultaneously and/or subsequently, by graft polymerization of monomer that provides hydrophilic group.

After uniformly-sized liquid drops are formed in dispersion medium by causing regular oscillation disturbance to liquid column of monomer mixture, spurted out into dispersion medium from a nozzle hole, polymerization is preferably conducted under the condition that neither connation nor additional dispersion of said liquid drops occurs.

BEST MODE FOR CARRYING OUT THE INVENTION

The invention relates to cross-linked polymer particle containing vinyl alcohol unit and nitrogen-containing polymerization unit as compounds, wherein nitrogen content in dry weight based on the total weight of said particle (hereinafter, simply called "nitrogen content"), determined by element analysis (CHN), is 7.0 to 13.0 weight %, wherein surface nitrogen content against said particle surface (hereinafter, simply called "surface nitrogen content"), determined by X-ray photoelectron spectroscopy (XPS), is 5.0 to 15.0 at%.

The polymerization unit used in the invention is a unit based upon monomers, provided for polymerization, and includes the unit derived from said unit through chemical reaction, etc. The nitrogen content in dry weight, determined by element analysis, is the nitrogen content of said cross-linked polymer particles in dry weight, determined by conducting element analysis by CHN recorder, etc. after substantially complete drying of sufficiently-washed said cross-linked polymer particles by vacuum dryer, etc., while the surface nitrogen content determined by X-ray photoelectron spectroscopy is the nitrogen concentration (at%: atomic percent) of said cross-linked polymer particles, determined by conducting surface element analysis by XPS on cross-linked polymer, substantially completely dried by t-butyl alcohol freeze-drying, whose particle structure in water is maintained.

In the cross-linked polymer particle containing alcohol unit and nitrogen-containing polymerization unit, the nitrogen content against the total weight of said particle in dry weight, determined by element analysis, is 7.0 to 13.0 weight %. Said nitrogen content is preferably at least 7.1 weight %, more preferably at least 7.3 weight %, further preferably at least 7.6 weight % and particularly preferably at least 7.8 weight %. The upper limit of the nitrogen content is preferably at most 12.0 weight %, more preferably at most 11.0 weight %, further preferably at most 10.5 weight %, particularly preferably at most 10.0 weight % and most preferably at most 9.5 weight %.

The surface nitrogen content (atomic concentration) against particle surface, determined by X-ray electron spectroscopy (XPS) on said cross-linked polymer particle, is 5.0 to 15.0 at%. The surface nitrogen content is preferably at least 6.0 at% and more preferably at least 7.0 at%; preferably at most 13.0 at%, more preferably at most 12.0 at%, particularly preferably at most 11.0 at% and most preferably at most 10.0 at%.

The difference between the percentage (weight %) of nitrogen content in dry weight, determined by element analysis, and the percentage (at%) of surface nitrogen content, determined by X-ray electron spectroscopy (XPS), in the cross-linked polymer particles in the invention is preferably less than 2.0 to suppress the activation of complement system and leukocytes at a particularly lower level; less than 1.5 is particularly preferred.

Less than 7.0 weight % of the nitrogen content in dry weight against the total weight of said particles, determined by element analysis, and less than 5.0 at% of surface nitrogen content determined by XPS are not preferred due to likely occurrence of activation of coagulation system, complement system and leukocytes. More than 13.0 weight % of nitrogen content in dry weight against the total weight of said particle, determined by element analysis, and more than 15.0 at% of surface nitrogen content, determined by XPS, are not preferred since the loss of useful substances is likely to increase due to nonspecific adsorption.

In view of the balance between the activation of coagulation system, complement system and leukocytes and nonspecific adsorption, the nitrogen content in dry weight, determined by element analysis, in cross-linked polymer particles consisting of synthetic polymer including vinyl alcohol unit and nitrogen-containing polymerization unit in the invention as components is 7.3 to 9.2 weight %. Said nitrogen content is preferably at least 7.5 weight %, more preferably at least 7.7 weight %, particularly preferably at least 7.9 weight % and most preferably at least 8.1 weight %. The upper limit of said nitrogen content is preferably at most 9.0 weight %, more preferably at most 8.8 weight %, particularly preferably at most 8.6 weight % and most preferably at most 8.4 weight %.

Furthermore, the cross-linked polymer particle containing vinyl alcohol unit and nitrogen-containing polymerization unit as components according to the present invention have preferably a percentage of nitrogen-containing polymerization unit against all the polymerization units that constitutes cross-linked particles of from 41.0 to 75.0 weight %, wherein percentage of nitrogen-containing polymerization unit on the surface of said cross-linked polymer particle is 30.0 to 85.0 weight %.

The percentage of nitrogen-containing polymerization unit against all the polymerization units that constitute said cross-linked polymer particles is preferably at least 42.0 weight %, more preferably at least 44.0 weight % and particularly preferably at least 46.0 weight %, and preferably at most 70.0 weight %, more preferably at most 65.0 weight %, particularly preferably at most 63.0 weight % and most preferably at most 60.0 weight %.

The percentage of nitrogen-containing polymerization unit on the surface of said particle is preferably at least 35.0 weight %, more preferably at least 40.0 weight %, and preferably at most 75.0 weight %, more preferably at most 70.0 weight %, particularly preferably at most 65.0 weight % and most preferably at most 60.0 weight %.

Less than 41.0 weight % of the nitrogen-containing polymerization unit against all the polymerization particles that constitute cross-linked polymer particles, and less than 30.0 weight % of nitrogen-containing polymerization unit on the surface of said particles are not preferred due to likely occurrence of activation of coagulation system, complement system and leukocytes. More than 75.0 weight % of nitrogen-containing polymerization unit against all the polymerization units that constitute cross-linked polymer particles, and more than 85.0 at% of nitrogen-containing polymerization unit on the surface of said particles are not preferred since the loss of useful substances is likely to increase due to nonspecific adsorption.

The difference between the percentage of nitrogen-containing polymerization unit against all the polymerization units that constitute cross-linked polymer particle and the percentage of nitrogen-containing polymerization unit on the surface of said cross-linked polymer particle is preferably less than 15.0 weight % to suppress the activation of complement system and leukocytes at a particularly lower level; less than 10.0 weight % is particularly preferred.

In view of the balance between the activation of coagulation system, complement system and leukocytes and nonspecific adsorption, the percentage of nitrogen-containing polymerization unit against all the polymerization units that constitute cross-linked polymer particles is preferably 43.0 to 54.0 weight % in cross-linked polymer particles made of synthetic polymer containing vinyl alcohol unit and nitrogen-containing polymerization unit in the invention as components. The percentage of said nitrogen-containing polymerization unit is preferably at least 45.0 weight %, particularly preferably at least 47.0 weight % and most preferably at least 48.0 weight %. The percentage of said nitrogen-containing polymerization unit is preferably at most 52.0 weight %, particularly preferably at most 51.0 weight % and most preferably at most 50.0 weight %.

Less than 7.3 weight % of said nitrogen content and less than 43.0 weight % of the percentage of said nitrogen-containing polymerization unit may cause the activation of coagulation system, complement system and leukocytes unless the percentages of surface nitrogen content and nitrogen-containing polymerization unit on the surface of particles are within the above mentioned range. More than 9.2 weight % of said nitrogen content and more than 54.0 weight % of said nitrogen-containing polymerization unit may increase the loss of useful substances due to nonspecific adsorption unless the percentages of surface nitrogen content and nitrogen-containing polymerization unit on the surface of particles are within the above mentioned range. Particularly, lower bulk specific gravity of particles tends to generate fine particles.

The cross-linked polymer particles in the invention is usually used in aqueous medium; the percentage of nitrogen-containing polymerization unit against all the polymerization units that constitute cross-linked polymer particles, described in the invention, indicates the percentage of nitrogen-containing polymerization unit in dry weight against all the polymerization units that constitute said cross-linked polymer particles.

In the cross-linked polymer particle of the invention, if the chemical structure of the polymerization units that constitute said cross-linked polymer particles is known, the percentage of nitrogen-containing polymerization units against all the polymerization units that constitute said cross-linked polymer particles can be calculated from the nitrogen content in dry weight, etc., determined by element analysis.

For example, even if specific polymerization unit is incorporated by copolymerization, the composition of the generated copolymer does not necessarily match the feed composition when the polymerization conversion rate is less than 100 %. Therefore, the percentage of said nitrogen content or said nitrogen-containing polymerization unit should be determined for those that are actually used as processing materials and carriers thereof.

In the cross-linked polymer particle in the invention, if the polymerization units that constitutes said cross-linked polymer particle is known, the percentage of nitrogen-containing polymerization units on the surface of said cross-linked polymer particles can be calculated from the surface nitrogen content, etc., determined by surface element analysis by XPS.

The polymer surface is known to be subject to various chemical and physical interface actions during the formation of said surface and thus the surface composition of copolymer does not necessarily match the average composition of whole particles. Therefore, the surface nitrogen content of said cross-linked polymer particle or the percentage of nitrogen-containing polymerization units on said surface of cross-liked polymer particle should be determined for those that are actually used as processing materials and carriers thereof.

As described above, suppressing the activation of complement system, leukocytes, etc. is a serious issue of adsorbents and carriers thereof for the processing of body fluid, etc.; the cross-linked polymer particles in the invention is considered to specifically reduce the interaction with complement system, leukocytes, etc. and reduce the activation of complement system, leukocytes, etc., when nitrogen exists in the molecule at a specific rate, or nitrogen-containing polymerization unit exists at a specific rate.

In addition, physiologically active substances and cells in processed liquid cause interaction on the surface of said cross-linked polymer particle and thus the properties of the surface of said particle have important implications. In the cross-linked polymer particle of the invention, limiting the abundance of nitrogen on the surface of said cross-linked polymer particle to a specified range, or limiting the abundance of nitrogen-containing polymerization unit on the surface of said cross-linked polymer particle to a specified range is considered to be able to further reduce the interaction with complement system, leukocytes, etc. and markedly reduce the activation of complement system, leukocytes, etc.

Nitrogen-containing polymerization unit can be incorporated, for example, by copolymerization of nitrogen-containing monomers.

From the aspect of the designing of polymer particle, the vinyl alcohol unit in the cross-linked polymer particle of the invention is preferably formed through polymerization of monomer mixture containing at least monomer that provides vinyl alcohol unit and nitrogen-containing monomer and subsequent formation of part or all of monomer units that provide vinyl alcohol unit through chemical reactions such as hydrolysis and/or ester exchange reaction. Said monomer mixture can contain other monomers, non-polymeric liquid, linear polymer, polymerization initiator, etc. as needed.

Carboxylic vinyl ester is preferable as monomer that provides vinyl alcohol unit. The carboxylic vinyl esters, preferably used in the invention, include aliphatic carboxylic vinyl ester compounds such as vinyl acetate, vinyl propionate, vinyl butyrate, vinyl valerate, vinyl caproate, divinyl adipate, and aromatic carboxylic vinyl ester compounds such as vinyl benzoate and vinyl phthalate as examples. Among them, from the aspect of propensity to polymerization and hydrolysis/ester exchange reaction and economic efficiency, aliphatic carboxylic vinyl ester compounds such as vinyl acetate and vinyl propionate are preferable, and vinyl acetate is most preferable. Carboxylic vinyl ester can be used either singly or in combination.

Monomer with triazine ring is preferable as the nitrogen-containing monomer of the invention since it suppressed the activation of complement system and leukocytes to a lower level; for example, vinyl compounds with triazine ring, such as triallyl cyanurate, triallyl isocyanurate and trimetaallyl isocyanurate are included. Isocyanurates such as triallyl isocyanurate and trimetaallyl isocyanurate are particularly preferable for their physical or chemical stability, and triallyl isocyanurate is most preferable for easiness in handling. Nitrogen-containing monomer can be used either singly or in combination.

In the present invention, besides the above mentioned monomers, monomer capable of direct or indirect copolymerization with the above mentioned monomers can be used optionally. In the manufacturing method of polymer particle of the invention, monomers other than the above mentioned monomers can also be used singly.

Said monomers include alkyl acrylates, such as acrylic acid, methyl acrylate, ethyl acrylate and butyl acrylate, acrylic acids and esters thereof, such as methoxyethyl acrylate, 2-hydroxyethyl acrylate, 2-hydroxypropyl acrylate, glycidyl acrylate, polyoxyethylene monoacrylate, polyoxypropylene monoacrylate, polyethylene glycol diacrylate, polyalkylene glycol diacrylate, 1.6-hexanediol diacrylate, trimethylolpropane triacrylate, pentaerythritol triacrylate, dipentaerythritol hexaacrylate, alkyl methacrylates, such as methacrylic acid, methyl methacrylate and butyl methacrylate, methacrylic acids and esters thereof, such as t-butyl methacrylate, methoxyethyl methacrylate, cyclohexyl methacrylate, benzyl methacrylate, 2-ethylhexyl methacrylate, 2-hydroxyethyl methacrylate, 2-hydroxypropyl methacrylate, glycidyl methacrylate, glycerol monomethacrylate, polyoxyethylene monomethacrylate, polyoxypropylene monomethacrylate, tetrahydrofurfuryl methacrylate, ethylene glycol dimethacrylate, diethylene glycol dimethacrylate, polyethylene glycol dimethacrylate, polyalkylene glycol dimethacrylate, 1.3-butylene glycol dimethacrylate, glycerol dimethacrylate, trimethylolpropane trimethacrylate, aromatic vinyl compounds, such as styrene, methylstyrene, ethylstyrene, stryrenesulfonate, vinylnaphthalene, vinylbiphenyl, 1,1-diphenylethylene, vinyl benzoate, ethylvinylbenzene, divinylbenzene, divinylnaphthalene, divinylbiphenyl and vinyl compounds, such as acrylonitrile and methacrylonitrile. The after-mentioned polyfunctional vinyl compounds can also be used. These compounds can be used either singly or in combination as needed.

The cross-linked structure in the cross-linked polymer particle of the invention can be obtained by, for example, copolymerization of polyfunctional vinyl compounds. The examples of polyfunctional vinyl compounds include acrylates and methacrylates, such as ethylene glycol dimethacrylate, diethylene glycol dimethacrylate, polyethylene glycol dimethacrylate, polyethylene glycol diacrylate, polyalkylene glycol diacrylate, polyalkylene glycol dimethacrylate, 1.3-butylene glycol dimethacrylate, 1.6-hexanediol diacrylate, glycerol dimethacrylate, trimethylolpropane triacrylate, trimethylolpropane trimethacrylate, pentaerythritol triacrylate and dipentaerythritol hexaacrylate, divinyl ethers such as butanediol divinyl ether and diethylene glycol divinyl ether, aromatics such as divinylbenzene and divinylnaphthalene, and allyl compounds such as allyl methacrylate, triallyl cyanurate, triallyl isocyanurate and diallyl phthalate. Particularly, from the aspect of high mechanical strength and easy obtaining of dense pore structure, compounds with at least trifunctional crosslinking vinyl, such as trimethylolpropane trimethacrylate, trimethylolpropane triacrylate, pentaerythritol triacrylate, dipentaerythritol hexa acrylate triallyl cyanurate, triallyl isocyanurate and trimetaallyl isocyanurate, are more preferable.

The cross-linked structure in the cross-linked polymer particle of the invention, formed with nitrogen-containing polymerization unit, is preferred, since it reduces the activation of complement system and leukocytes to a lower level. Thus, polyfunctional vinyl compounds with triazine ring, such as triallyl cyanurate, triallyl isocyanurate and trimetaallyl isocyanurate, are particularly preferred, and triallyl isocyanurate is most preferable. These cross-linking monomers can be used either singly or in combination.

Generally, when polymerization conversion rate is high enough in copolymerization reaction, average composition of the finally-obtained copolymer matches the composition of feed monomer. The composition of copolymer, generated at a certain time point, is known to be subject to the effects of the monomer composition at that time and different reactivity of each monomer against a certain radical. Therefore, the composition of copolymer generated at a certain time point does not necessarily matches the composition of feed monomer. Specifically, the composition and composition distribution of copolymer obtained after polymerization at a relatively low polymerization conversion rate may differ widely from those of copolymer obtained at a high polymerization conversion rate that is generally used in usual copolymerization. In the cross-linked polymer particle of the invention, the properties such as composition distribution of said cross-linked polymer particle can be made more preferable in the affinity for physiologically active substance by adjusting the combination of monomers and the polymerization conversion rate.

Specifically, in the cross-linked polymer particle of the invention, for the polymerization of monomer mixture including at least monomer containing carboxylic vinyl ester and triazine ring, it is preferable that polymerization is conducted at 10 to 80 % of the polymerization conversion rate of carboxylic vinyl ester, cross-linked polymer containing carboxylic vinyl ester unit as a component is formed, and subsequently hydrolysis and/or ester conversion reaction are carried out to generate cross-linked polymer particle with part or all of carboxylic vinyl ester units being vinyl alcohol unit, since the activation of coagulation system, complement system and leukocytes is further reduced.

The polymerization conversion rate of carboxylic vinyl ester is preferably 10 to 70 %, more preferably 15 to 65 %, particularly preferably 20 to 60 % and most preferably 25 to 55 %.

When the polymerization conversion rate of carboxylic vinyl ester is low (less than 10 %), it is difficult to achieve sufficient particle strength. High polymerization conversion rate of carboxylic vinyl ester (more than 80 %) is not preferred since it tends to increase the activation of coagulation system, complement system and leukocytes.

It is preferable that the nitrogen content of said cross-linked polymer particle in dry weight and the percentage of nitrogen-containing particle unit against all the polymerization units that constitute said cross-linked polymer particle are higher than the nitrogen content and percentage of nitrogen-containing polymerization unit that are calculated from the composition of feed monomer, since the activation of coagulation system, complement system and cells is further reduced.

Said nitrogen content in dry weight is preferably at least 0.5 weight %, more preferably at least 1 weight % and most preferably at least 1.5 weight %, compared with the nitrogen content calculated from the feed monomer composition.

Said percentage of nitrogen-containing polymerization unit is preferably at most 5 weight %, more preferably at most 7 weight % and most preferably at most 10 weight %, compared with the percentage of nitrogen-containing polymerization unit calculated from the feed monomer composition.

In the polymerization of monomer mixture containing at least monomer containing carboxylic vinyl ester and vinyl compound having triazine ring, 10 to 200 parts by weight of monomers with triazine ring is preferably used against 100 parts by weight of carboxylic vinyl ester; more preferably 20 to 120 parts by weight, particularly preferably 20 to 80 parts by weight and most preferably 24 to 60 parts by weight.

In view of the balance between the activation of coagulation system, complement system and leukocytes and nonspecific adsorption, in the polymerization of monomer mixture containing vinyl compounds containing carboxylic vinyl ester and vinyl compound having triazine ring, 20 to 50 parts by weight of vinyl compound having triazine ring is preferably used against 100 parts by weight of monomer containing carboxylic vinyl ester; particularly preferably 20 to 40 parts by weight and most preferably 24 to 34 parts by weight.

Small amounts of vinyl compound having triazine ring (i.e., less than 10 parts by weight against 100 parts by weight of monomer containing carboxylic vinyl ester) tends to increase the activation of complement system and leukocytes. Large amounts of vinyl compound having triazine ring (i.e., more than 200 parts by weight) tends to increase nonspecific adsorption and adhesion of platelets.

The polymer particle of the invention is preferably polymer particle having pore structure, obtained using monomer mixture in which at least 10 weight % of all monomers are cross-linking monomers. The usage of cross-linking monomer is more preferably at least 15 weight % of all monomers in monomer mixture, and particularly preferably at least 20 weight %. The usage of cross-linking monomer is preferably at most 80 weight % of all monomers in monomer mixture, more preferably at most 60 weight %, and particularly preferably at most 40 weight %. When polymer particle has pore structure, particle strength is subject to the effects of the usage of cross-linking monomer. When the usage of cross-linking monomer is less than 10 weight % of all monomers included in the monomer mixture, polymer particle having pore structure, in particular, may lack mechanical strength. Particularly when bulk specific gravity of particle is low, excessive usage of cross-linking monomer may weaken the particle, and part of the particles are subject to destruction and chipped off to generate fine particles.

The volume-weighted mean diameter of the polymer particle of the invention is 50 to 3,000 $\mu$m; at least 80 volume % of said particles are preferably cross-linked polymer particles of 0.8 to 1.2-fold volume-weighted mean diameter.

The preferable volume-weighted mean diameter of said particles is at least 100 $\mu$m. When processed liquid contains cell components such as blood, or the processed liquid is highly viscous, at least 150 $\mu$m is preferable; more preferably at least 220 $\mu$m, particularly preferably at least 300 $\mu$m and most preferably at least 400 $\mu$m. The volume-weighted mean diameter of the cross-linked polymer particle of the invention is at most 2,000 $\mu$m, more preferably at most 1,500 $\mu$m, particularly preferably at most 1,000 $\mu$m, most preferably at most 710 $\mu$m.

Small volume-weighted mean diameter of less than 50 $\mu$m tends to narrow the interparticle space that will be a passage of the processed liquid, increase the pressure loss, cause nonspecific activation of cells, reduce the passage of cells and cause column clogging. Volume-weighted mean diameter of more than 3,000 $\mu$m tends to widen the interparticle space that will be a passage of the processed liquid, reduce the pressure loss and be advantageous to passage, but become less efficient as adsorbent and processing material.

When processed liquid contains cell components such as blood, or the processed liquid is highly viscous, particles of less than 100 $\mu$m preferably account for at most 5 volume %. More preferably, particles of less than 100 $\mu$m account for at most 1 volume %, more preferably at most 0.1 volume %, especially preferably at most 0.01 volume %, particularly preferably at most 0.001 volume %; most preferably, particles of less than 100 $\mu$m are not substantially included.

When particles of at most 100 $\mu$m exist at more than 5 volume %, small particles may fill interparticle space, increase pressure loss, cause nonspecific activation of cells, reduce the passage of cells and cause column clogging. A concern about the outflow of fine particles increases during use.

The volume elasticity of the cross-linked polymer particle of the invention, filled in a cylinder to be pressured by a piston in water, is preferably 0.02 to 2 MPa. The volume elasticity is more preferably 0.1 to 1 MPa, more preferably 0.12 to 0.8 MPa, particularly preferably 0.15 to 0.6 MPa and most preferably 0.15 to 0.35 MPa. Less than 0.02 MPa of volume elasticity is likely to deform particles, reduce the interparticle space that will be a passage, increase the pressure loss and cause column clogging. In addition, it is not preferred since fine particles are likely to be generated due to insufficient strength. Although particles are hard and undeformable, more than 2 MPa of volume elasticity is not preferred since activation of cells and nonspecific adhesion to particles are likely to occur by the stimuli such as impact when cells contact with particles at high speed during the flow of cell-containing liquid, such as blood, lymph fluid and spinal fluid.

The invention is also related to body fluid processing material consisting of said cross-linked polymer particles.

In the general suspension polymerization, liquid drops of monomer mixture are generated in dispersion medium by mechanical shaking using a stirring blade, during which the liquid drops are divided to be smaller or combined with other liquid drops to be larger. Thus, the obtained polymer particles have wide particle size distribution containing large and small particles. The particles with wide particle size distribution, obtained by suspension polymerization, etc., are preferably used after the large and small particles are removed by classification procedures such as screening.

In addition, the invention provides a manufacturing method of uniformly-sized polymer particles, including a process (a) to form uniformly-sized liquid drops of monomer mixture in dispersion medium and a process (b) for polymerization under the condition that neither connation nor additional dispersion of said liquid drops occurs.

The condition that neither connation nor additional dispersion of said liquid drops occurs, used in the invention, means the condition that drops are generally not combined with other liquid drops to be larger (connation) or not divided to be smaller (additional dispersion). In the invention, to form uniformly-sized liquid particles of monomer mixture in dispersion medium, when said monomer mixture can form liquid drops, part of monomers may be polymerized in advance, or part of monomers can be polymerized simultaneously with liquid drop formation. The above mentioned "uniformly size" means that most particles have almost comparable size, and large and small particles are not included.

The volume-weighted mean diameter of the particles, provided by the manufacturing method of the invention, is 50 to 3,000 $\mu$m; at least 80 volume % of said particles are polymer particles with 0.8 to 1.2-fold volume-weighted mean diameter. And preferably, the volume-weighted mean diameter of the polymer particles is 150 to 3,000 $\mu$m; particles with less than 100 $\mu$m of particle size account for at most 5 volume %.

At least 80 volume % of said particles preferably have 0.9 to 1.1-fold volume-weighted mean diameter, at least 90 volume % of said particles more preferably have 0.9 to 1.1-fold volume-weighted mean diameter, at least 95 volume % of said particles particularly preferably have 0.9 to 1.1-fold volume-weighted mean diameter, and particles most preferably have substantially single particle size.

Even if the volume-weighted mean diameter is the same, wide particle size distribution (i.e., small particles of less than 0.8-fold volume-weighted mean diameter and large particles of more than 1.2-fold volume-weighted mean diameter account f than 20 volume %) tends to narrow the interparticle space that will be a passage of the processed liquid and increase the pressure loss during liquid feeding. Cell components included in the processed liquid may cause nonspecific activation of cells, reduce the passage of cells and cause column clogging.

The above mentioned process (a) is a process to form uniformly-sized liquid drops in dispersion medium. An example of liquid drop generator is an instrument, into which a nozzle to spray monomer mixture to a column filled with dispersion medium is inserted (the nozzle has at least one small opening), in which oscillation transmission mechanism is installed. A tube to supply dispersion medium from a dispersion tank can be connected to a column, and a tube to supply monomer mixture from a monomer mixture tank can be connected to the nozzle.

The particle size of uniformly-sized liquid drops, formed in the above mentioned process (a), is determined by factors, including physical and chemical properties such as the diameter of nozzle opening, passage time of monomer mixture through the nozzle and viscosity of monomer mixture and dispersion medium, and the type of oscillation disturbance generated by the passage of monomer mixture through the nozzle and given to the liquid column, frequency and amplitude; liquid drops of desired particle size can be obtained by these factors and combinations thereof. In addition, one or several factors of them can be changed in combination to obtain a liquid drop group with desired particle size distribution.

The nozzle used in the process (a) is not particularly limited; for example, orifice, etc. with at least one small opening can be used. Said nozzle can be used either singly or in combination.

Examples of providing oscillation disturbance include a method to spray monomer mixture from the nozzle while adding mechanical oscillation to the monomer mixture, a method to spray monomer mixture from the nozzle while adding mechanical oscillation to dispersion medium and a method to add mechanical oscillation to the nozzle to spray monomer mixture. Among them, although not limited to this, the method to spray monomer mixture from the nozzle while adding mechanical oscillation to the monomer mixture is efficient and preferred.

To obtain more uniformly-sized polymer particles, more uniformly-sized liquid drops should be formed; for this purpose, it is preferable to examine and select optimal condition associated with the above mentioned factors. In addition, it is preferable that the oscillation disturbance given to the liquid column has small variation in its frequency and amplitude.

In addition, it is preferable that the supply of monomer mixture has small variation in its pulse and flow rate; for example, multiple plunger pump, gear pump, etc. can be used as feeders.

Monomer mixture preferably contains at least one kind of monomer, selected from the group consisting of carboxylic vinyl ester, methacrylate ester, acrylic ester, aromatic vinyl compound and vinyl cyanide compound, and when multiple kinds are used, any combinations serve the purpose.

The above mentioned process (b) is a process to polymerize formed uniformly-sized liquid drops under the condition that neither connation nor additional dispersion occurs. The condition that neigher connation nor additional dispersion occurs, used in the invention, mean the condition that drops of monomer mixture, formed when oscillation disturbance is given by squirting from nozzle opening into dispersion medium, are substantially not combined with other liquid drops to be larger or not divided to be smaller.

For example, a method to lead the liquid drops, formed in the first process, into the reactor and to induce polymerization reaction while gently stirring the dispersion medium containing liquid drops can be employed as a method to conduct process (b). However, insufficient stirring tends to cause connation of liquid drops, and in contrast, excessive stirring tends to cause additional dispersion and thus the stirring method and its condition should be set as needed. For example, when a stirring blade is used for stirring, it is preferable that a type with relatively low shearing force and high discharge is selected and stirring speed is adjusted before use.

To reduce the connation of liquid drops and additional dispersion, liquid drop group is preferably led into the dispersion medium that preexists in a polymerization reactor, and liquid drop generator is preferably connected to the lower part of the polymerization reactor via a tube, etc. The processes (a) and (b) can be conducted by separate instruments, or by using a single instrument.

Dispersion medium, not mixable with monomer mixture that form liquid drops, is used to generate a continuous phase; aqueous medium is preferably used from the aspects of safety, economic efficiency and environment.

Said dispersion medium can contain dispersion stabilizer. No particular limitation is imposed upon the dispersion stabilizer, but those that are generally used for suspension polymerization can be used; for example, dispersion stabilizer soluble into dispersion medium, dispersion stabilizer for solid particulate, surfactant, etc. can be used.

These can be used singly, but when used in combination, they can have a profound effect. An auxiliary substance can be used in combination. Insufficient dispersion stabilizer tends to cause connation of liquid drops, and in contrast, excessive dispersion stabilizer tends to cause additional dispersion and thus care must be taken for the types and usage of dispersion stabilizer and auxiliary substance.

Polymer protective colloid is preferably used as a dispersion stabilizer soluble in dispersion medium. Specific examples include water-soluble polymers such as polyvinyl alcohol, polyacrylamide, polyvinyl pyrrolidone and sodium polyacrylate, water-soluble cellulose derivatives such as methyl cellulose, carboxymethyl cellulose and hydroxyethyl cellulose, and water-soluble natural polymers such as starch and gelatin. These can be used either singly or in combination.

The usage of dispersion stabilizer soluble in dispersion medium is preferably 0.001 to 10 weight % against dispersion medium, more preferably 0.005 to 5 weight %, further preferably 0.01 to 1.5 weight %, particularly preferably 0.015 to 0.6 weight % and most preferably 0.03 to 0.3 weight %.

More than 10 weight % of the usage of dispersion stabilizer that is soluble in dispersion medium is not preferred, since it tends to cause additional dispersion of liquid drops or generation of new particles through emulsion polymerization, etc.

Dispersion stabilizers for solid particulate include hardly-soluble inorganic substances such as calcium phosphate, calcium carbonate, magnesium hydroxide and magnesium pyrophosphate; tribasic calcium phosphate is particularly effective. These can be used either singly or in combination. The mean particle size of dispersion stabilizer for solid is preferably at most 50 $\mu$m, and more preferably at most 10 $\mu$m. The usage of dispersion stabilizer for solid particulate is preferably 0.01 to 20 weight % against dispersion medium, more preferably 0.05 to 10 weight %, particularly preferably 0.1 to 5 weight % and most preferably 0.3 to 3 weight %. Appropriate use of dispersion stabilizer for solid particulate has a profound effect on the prevention of secondary coagulation of polymerized particles.

Examples of surfactant include nonionic surfactants, such as polyoxyethylene alkyl ether, polyoxyethylene alkylarylether, polyoxyethylene sorbitan aliphatic ester, polyoxyethylene aliphatic ester, polyoxyethylene derivative, sorbitan aliphatic ester and glycerine aliphatic ester, and anionic surfactants, such as alkyl sulfate ester, alkyl benzene sulphonate, alkylnaphthalenesulfonate, $\alpha$-olefin sulphonate, dialkyl sulfosuccinate, alkyl-diphenyl-ether-sulphonate, alkyl phosphate, polyoxyethylene alkyl phosphate ester, polyoxyethylene alkyl sulfate ester and polyoxyethylene alkyl aryl sulfate ester. These surfactants can be used either singly or in combination. Concomitant use of these surfactants with said dispersion stabilizer can further improve the dispersion stability. Particularly when dispersion stabilizer for particulate solid is used, concomitant use of anionic surfactant is effective.

The usage of surfactant is preferably 0.0001 to 5 weight % against dispersion medium, more preferably 0.0005 to 0.5 weight %, particularly preferably 0.001 to 0.1 weight % and most preferably 0.002 to 0.05 weight %. More than 5 weight % of the usage of surfactant is not preferred since it tends to cause additional dispersion of liquid drops or generation of new particles through emulsion polymerization, etc.

Salts include chloride salts such as sodium chloride and potassium chloride, sulfate salts such as sodium sulfate and magnesium sulfate and phosphates such as sodium dihydrogen phosphate and disodium hydrogen phosphate.

The more preferable use of dispersion stabilizer is a method in which liquid drops of monomer mixture are formed in dispersion medium in the presence of dispersion stabilizer solution in dispersion medium, and dispersion stabilizer for particulate solid is added and/or said liquid drops are polymerized in the presence of salt, to form liquid drops of monomer mixture in dispersion medium.

By conducting said polymerization in the presence of polymerization inhibitor that is soluble in dispersion medium, generation of fine particles can be suppressed. Examples of polymerization inhibitors soluble in dispersion medium include nitrite salts such as sodium nitrite, hydroquinone and p-diaminophenylene. The concentration of polymerization inhibitor is preferably 0.0001 to 1 weight % against dispersion medium, more preferably 0.0005 to 0.5 weight %, particularly preferably 0.001 to 0.1 weight % and most preferably 0.002 to 0.05 weight %. More than 1 weight % of the usage of polymerization inhibitor is not preferred since it inhibits the smooth progress of polymerization reaction.

The manufacturing method of the invention more preferably include process (c) for further processing and modification after said processes (a) and (b).

The processing and modification in said process (c) are conducted for the improvement of hydrophilicity, biocompatibility, physical and chemical properties and mechanical strength of polymer particles, functionalization as processing material, reduction of eluate, sterilization, etc. Examples of processing and modification include incorporation of hydroxyl group and other functional groups, hydrolysis and ester exchange reaction, ligand immobilization, coating and mutiphase formation, crosslinking, polymerization reactions such as graft polymerization, washing, high-temperature treatment and high-pressure treatment, radiation treatment, plasma treatment, other chemical and/or physical processing and modification, etc., which can be conducted simultaneously with and/or after polymerization.

The polymer particles obtained by the manufacturing method of the invention can have pore structure. The pore structure can markedly increase the contact area with processed liquid and process object substance more efficiently.

In the manufacturing method of polymer particle of the invention, vinyl alcohol unit is preferably obtained through a process to polymerize monomer mixture containing carboxylic vinyl ester and polyfunctional vinyl compound to form cross-linked polymer containing carboxylic vinyl ester unit as one of the components and a process to turn part or all of said carboxylic vinyl ester units into vinyl alcohol unit through hydrolysis and/or ester exchange reaction. The compounds in said cross-linked polymers are included as carboxylic vinyl ester and polyfunctional vinyl compound, which can be obtained through a process to form cross-linked polymer, and as hydrolysis and/or ester exchange reaction, by the same method as that to obtain vinyl alcohol unit in said cross-linked polymer particles.

The manufacturing method of polymer particle of the invention preferably includes a process to modify particle surface through polymerization of monomer mixture containing at least one kind of monomer, selected from the group consisting of carboxylic vinyl ester, methacrylate ester, acryl ester, aromatic vinyl compound and vinyl cyanide compound, as a major monomer, and simultaneously and/or subsequently, graft polymerization of monomers that provide hydrophilic group to the polymer particles. Particularly when monomers that provide hydrophilic group are graft-polymerized in dispersion medium, effective modification of polymer surface with hydrophilic group is preferred. More preferably, hydrophilic dispersion medium such as water and monomer with hydrophilic group soluble in said dispersion medium are used to graft-polymerize monomers with hydrophilic group into polymer particles in said dispersion medium.

Monomers that provide hydrophilic group include monomer having hydrophilic group and monomer that may generate hydrophilic group through chemical reactions such as hydrolysis, ester exchange reaction and ring-opening.

Examples of monomer that may generate hydrophilic group include alkyl acrylates such as carboxylic vinyl ester, methyl acrylate, ethyl acrylate and butyl acrylate, acryl esters such as methoxyethyl acrylate, alkyl methacrylates such as methyl methacrylate and butyl methacrylate, vinyl compounds with ester unit such as methacrylic esters, including t-butyl methacrylate, methoxyethyl methacrylate, cyclohexyl methacrylate, benzyl methacrylate, 2-ethylhexyl methacrylate and tetrahydrofurfuryl methacrylate, and vinyl compound with glycidyl such as glycidyl methacrylate, glycidyl acrylate and allyl glycidyl ether. These can be used either singly or in combination. Carboxylic vinyl ester and methacrylate ester are most preferable.

Examples of monomer with hydrophilic group include vinyl compounds with carboxyl group, such as acrylic acid and mathacrylic acid, vinyl compounds with hydroxyl group, such as 2-hydroxyethyl acrylate, 2-hydroxypropyl acrylate, 2-hydroxyethyl methacrylate (HEMA), 2-hydroxypropyl methacrylate and glycerin monomethacrylate, vinyl compounds with polyoxyethylene, such as polyethylene glycol monoacrylate and polyethylene glycol monomethacrylate, vinyl compounds with polyoxypropylene group, such as polypropylene glycol monoacrylate and polypropylene glycol monomethacrylate, vinyl compounds with pyrrolidone group, such as N-vinyl pyrrolidone and vinyl compounds with amide group, such as acrylamide and methacrylamide. These can be used either singly or in combination.

Among the abovementioned vinyl compounds, in terms of possible modification effect, vinyl compounds having hydroxyl, polyoxyethylene or pyrrolidone group are preferable, and those having hydroxyl or polyoxyethylene group are more preferable, and those with hydroxyl group are most preferable.

Graft polymerization of monomers that provide hydrophilic group can also be conducted either by adding polymerization initiator to dispersion medium (i.e., continuous phase) or with polymerization initiator added to monomer mixture (i.e., dispersion phase). Also oxidant and reducer can be used to make a redox system; as for others, auxiliary agent, etc. can also be used as needed. Examples of polymerization initiators, soluble in hydrophilic dispersion medium, include persulfates such as potassium persulfate and sodium persulfate and heretofore known water-soluble polymerization initiators such as hydrogen peroxide and hydroperoxides. In addition, methods using ultraviolet, radiation, plasma, etc. can be used. These can be used either singly or in combination.

For the polymerization reaction of polymer particle of the invention, all the heretofore known methods can be used; however, radical polymerization is preferred due to its economic efficiency and easiness in polymerization, and a method by heat degradation of polymerization initiators, use of a redox system, photo polymerization and ultraviolet polymerization, radiation polymerization and other heretofore known methods can be used. Polymerization initiator, catalyzer, chain transfer agent and other auxiliary agents can be selected and used as needed.

For example, in the method by heat degradation of polymerization initiator, examples of polymerization initiators include, but are not limited to, azo compounds such as 2,2'-azobis (isobutyronitrile), 2,2'-azobis (2,4-dimethylvaleronitryl), 2,2'-azobis (2-methylbutyronitrile), 2,2'-azobis (2-methylpropionamidine) dihydrochloride, and organic peroxides such as benzoyl peroxide, lauroyl peroxide, t-butyl perbenzoate, t-butyl perpivalate, t-butyl peroxy isopropylcarbonate, t-butyl peroxyacetate and t-butyl peroxy-3,3,5-trimethylcyclohexane. Photo polymerization initiator can be used for photo polymerization, etc.

The polymerization initiator used in the polymerization of monomer mixture is preferably soluble in monomer mixture for the promotion of smooth progress of polymerization reaction. In addition, polymerization initiators, insoluble or sparingly soluble in dispersion medium, are preferred, since they generate only a small amount of fine particles.

In the invention, the usage and kind of the polymerization initiators are preferably selected to achieve predetermined polymerization conversion rate in consideration of properties as polymerization initiator, such as reactivity with monomer and degradation rate, and reaction conditions such as polymerization time and polymerization temperature; the polymerization initiators can be used singly or in combination. From the aspect of the control of polymerization reaction, the polymerization temperature is preferably 0 to 180°C, more preferably 20 to 120°C, further preferably 40 to 90°C and most preferably 50 to 70°C.

In the polymer particle of the invention, non-polymeric liquid can be used in monomer mixture for the incorporation of pore structure, etc. Use of pore structure markedly increases the contact area with processed liquid and is advantageous to the improvement of processing performance. In this case, optimal pore size and pore structure can be selected as needed according to the size and shape of processed substance. From the aspect of processing performance, it is preferable to provide pore size and pore structure that allow easy passage of processed substance and make the accessible area with the processed substance as large as possible. Selection of pore size can provide selectivity based upon molecular-sieve effects such as adsorption/removal of only substances that may enter pore.

Body fluid processor that can selectively remove or collect specific cells from liquid containing cells, such as blood, lymph fluid, spinal fluid, etc. and have some favorable effects on specific cells is expected to be applied to new treatment methods such as leukocyte apheresis and cell treatment, however, adsorbent, processing material and carriers thereof for such cells do not necessarily have to be porous.

To obtain uniform pore structure, it is preferred that non-polymeric liquid is soluble in monomer mixture, and is insoluble or sparingly soluble in dispersion medium.

Specific examples include, but are not limited to, aromatic hydrocarbons such as toluene, xylene and benzene, aliphatic hydrocarbons such as hexane, heptane, pentane and octane, ester compounds such as ethyl acetate, n-butyl acetate and n-hexyl acetate, ethers such as dibutyl ether, alcohols such as n-heptanol and amyl alcohol and ketones such as methyl isobutyl ketone.

With the progress of polymerization, non-polymeric liquid and residual monomers undergo phase separation in the generated cross-linked polymer and particles and form pore region. Therefore, the structure of the pore region is influenced by the usage of non-polymeric liquid and the difference of affinity between non-polymeric liquid and the generated cross-linked polymer. For example, selection of non-polymeric polymer with lower affinity with the generated cross-linked polymer tends to increase the pore size of the obtained polymer particles.

Non-polymeric liquid can be used either singly or in combination; its kind and mixing ratio can change the affinity with the generated polymer and adjust pore structure. In addition, pore structure, including pore volume, can be adjusted by changing the usage of non-polymeric liquid.

The usage of non-polymeric liquid is preferably at most 400 parts by weight, more preferably at most 300 parts by weight, particularly preferably at most 250 parts by weight and most preferably at most 200 parts by weight. More than 400 parts by weight of the usage of non-polymeric liquid tends to cause insufficient strength.

When the incorporation of pore structure into the polymer particle of the invention is needed, the usage of non-polymeric liquid is preferably at least 50 parts by weight, more preferably at least 100 parts by weight, particularly preferably at least 130 parts by weight and most preferably at least 150 parts by weight.

For the polymer particle of the invention, linear polymer can be used for the incorporation of pore structure, etc. The linear polymer used in the invention indicates substantially uncross-linked polymer, which can include branch structure, and if soluble in medium, some cross-linked structure. The degree of polymerization and usage of linear polymer have a profound effect on the structure of porous region. For example, increasing the usage of linear polymer and the average degree of polymerization tends to increase the pore size of the obtained polymer particle. Use of non-polymeric liquid and linear polymer in combination allows easy control of the pore structure of cross-linked polymer particle.

Specific examples of linear polymer include ester resins such as polyvinyl acetate, acrylic resins such as polymethylmethacrylate and polyacrylonitrile, aromatic resins such as polystyrene, halogen resins such as polyvinyl chloride, diene resins such as polybutadiene and copolymers and blends thereof. Other polymers can be used; there is no particular limitation on polymers as far as they are soluble in monomer mixture.

The usage of linear polymer is preferably at most 100 parts by weight, more preferably at most 40 parts by weight, particularly preferably at most 20 parts by weight and most preferably at most 15 parts by weight.

When pore structure has to be incorporated into the polymer particle of the invention, the usage of linear polymer is preferably at least 1 parts by weight, more preferably at least 1 parts by weight, particularly preferably at least 2 parts by weight and most preferably at least 3 parts by weight.

The average degree of polymerization of linear polymer is preferably 100 to 10,000, more preferably 100 to 5,000, further preferably 150 to 3,000, particularly preferably 200 to 1,500 most preferably 300 to 1,000.

More than 100 parts by weight of the usage of linear polymer and more than 10,000 parts by weight of the average degree of polymerization cause high viscosity of monomer mixture and difficulty in handling. Aggregate tends to be generated during polymerization.

After uniformly-sized liquid drops are formed in dispersion medium by causing regular oscillation disturbance to liquid column of monomer mixture, spurted out into dispersion medium from a nozzle hole, said liquid drops can be polymerized under the condition that neither connation nor additional dispersion occurs, and undergo processing and modification as needed. This allows obtainment of uniformly-sized said polymer particles that have been difficult to be obtained by the conventional method. The polymer particle of the invention, thus obtained, has advantages of the minimal presence of large and small particles and marked reduction of classification loss.

The polymer particles, generated by the general suspension polymerization, are formed through the progress of polymerization while involving dispersion medium and dispersion stabilizer into the particles when liquid drops are divided or combined with other liquid drops by stirring. On the other hand, according to the manufacturing method of the invention, after uniformly-sized liquid drops of monomer mixture are formed in dispersion medium, said liquid drops are polymerized under the condition that neither connation nor additional dispersion occurs, and said liquid drops are characterized in that they are formed by the progress of polymerization without involving dispersion medium and dispersion stabilizer into the particles.

In the manufacturing method of polymer particle of the invention, besides the abovementioned method using crosslinking monomer, the crosslinking structure can be incorporated by the heretofore known methods such as a method using reactive functional group or binding functional group, method by adding crosslinking agent or coagent, peroxide crosslinking, photo crosslinking, radiation crosslinking, electron crosslinking and polymer reaction; a method using crosslinking polymer is preferred from the aspect of the easiness in designing cross-inking structure.

The polymer particle of the invention can be used without change or as adsorbent or processing material after incorporating a substance that can bind to a component or cells to be adsorbed and/or a substance that favorably interacts with physiologically active substance or cells, as ligand. For the incorporation of ligand, hydroxyl group in vinyl alcohol unit and ester in carboxylic vinyl ester unit can be used either directly or indirectly. The cross-linked polymer particle of the invention can be used by being mixed with at least two different said polymer particles or with said polymer particles of different average particle size.

The polymer particle of the invention can be used in methods such as separating processed liquid from said polymer particles by filtration, centrifugation, etc. after they are mixed and contacted with processed liquid in a container. More preferably, the polymer particle of the invention can be used in a method in which they are filled into a container with inlet and outlet, like column, and processed liquid is flown in from the inlet to allow the contact of said polymer particles with the processed liquid in the container, and the processed liquid is let out from the outlet. In said container, structures such as mesh that allows the passage of said polymer particles but not processed liquid can be set.

EXAMPLES

EXAMPLE 1

(Formation of uniform liquid drops)

438.2 g of water, 1.83 g of 3 weight % aqueous solution of sodium α-olefin sulphonate and 126.4 g of 10 weight % slurry of particulate tribasic calcium phosphate were fed into a 2 L separable flask with a stirring blade and gently mixed.

An instrument, in which a nozzle to spray monomer mixture to a column filled with dispersion medium is inserted, the upper part of the nozzle is made of an orifice plate with at least 12 small openings of 0.17 mm pore size, an oscillation plate to transmit oscillation is set at the lower part, and the oscillation plate is connected to oscillation exciter, was used as liquid drop generator. A tube to supply dispersion medium from a dispersion medium tank was connected to a column, and a tube to supply monomer mixture from a monomer mixture tank was connected to the nozzle. A double plunger pump with high quantitative capability and less pulsing was used for the supply of monomer mixture.

523.6 g of monomer mixture (composition in Table 1), consisting of 100 parts by weight of vinyl acetate, 26 parts by weight of triallyl isocyanurate (TAIC), 122 parts by weight of ethyl acetate, 51 parts by weight of heptane, 12.8 parts by weight of polyvinyl acetate (PVAc) (average degree of polymerization: 400) and 5.1 parts by weight of 2,2'-azobis (2,4-dimethyl valeronitrile) (V-65), was supplied from a monomer mixture tank to nozzle at a rate of 27.6 mL/min and fed into a separable flask via liquid drop generator. Simultaneously, 610.8 g of dispersion medium, prepared with 83.4 g of 3 weight % polyvinyl alcohol (PVA) aqueous solution, 4.62 g of 6 weight % sodium nitrite aqueous solution and 2,412 g of water, was supplied from a dispersion medium tank to the column and fed into the separable flask via the liquid drop generator.

At this time, mechanical oscillation at constant frequency (500 Hz) and intensity of 0.4 G was provided to monomer mixture by oscillation exciter. The liquid column of monomer mixture, spurted out from the nozzle opening, was divided by said mechanical oscillation to form uniformly-sized liquid drops in dispersion medium in the column. The formed liquid drops were sent into a separable flask together with dispersion medium that was supplied simultaneously.

(Polymerization)

Subsequently, the content of the separable flask was stored at 65°C for 5 hours under a nitrogen atmosphere to polymerize said liquid drops. Said liquid drops were sampled at each time point of column outlet, before polymerization and after polymerization, and were observed by a digital fine scope (manufactured by Omron Corp. 3D digital fine scope VC1000). All of said liquid drops maintained uniform size, demonstrating that polymerization was carried out without connation and further dispersion. The obtained polymerized slurry was sampled and weighed, and after addition of polymerization inhibitor (hydroquinone), the volatile portion was dried in an oven at 120°C, and the constant mass was confirmed and dry weight was measured. Only a small quantity of TAIC was lost under said dry condition, and the polymerization conversion rate of vinyl acetate was calculated as 51 % from the weights before and after drying.

(Post-processing)

Subsequently, hydrochloric acid was added to the content in the separable flask to adjust the pH to at most 2 to dissolve tribasic calcium phosphate, followed by thorough washing with water. After confirming that the pH of the washing reached around neutral pH, the water was replaced by acetone, and the polymer was thoroughly washed with acetone. After the acetone was replaced by water, the quantity of sodium hydroxide (NaOH), calculated by the following formula, was added in excess to vinyl acetate unit:

$$\text{NaOH (solid content weight)} = \text{dry weight of particles}/86.09$$

$$\times\ 40 \times 1.5$$

The volume of water was adjusted to make the NaOH concentration against water to be 4 weight %. This was incubated at reaction temperature of 40°C for 6 hours with stirring to be saponified. Subsequently, this was washed with water until the pH reached around neutral pH and was thoroughly washed with hot water at 80°C. Subsequently, autoclaving was conducted at 121°C for 20 minutes, and clean cross-linked polymer particles were obtained.

(Measurement of particle size)

Said cross-linked polymer particles in water were photographed by said digital fine scope. The particle size was measured based upon the obtained images; the number-average particle size was 459 $\mu$m, and the volume-average particle size was 469 $\mu$m. Particles of 0.8 to 1.2-fold volume-average particle size accounted for 99.3 volume %, particles of 0.9 to 1.1-fold volume-average particle size accounted for 98.0 volume %, and particles of less than 100 $\mu$m accounted for 0.006 volume %. The results are shown in Table 2.

(Bulk specific gravity)

The dry solid weight per 1 mL sedimentation volume of the particles, uniformly spun down in water by tapping, etc., (bulk specific gravity) was 0.134 g/mL.

(SEM observation)

To observe the obtained cross-linked polymer particles by scanning electron microscope (SEM) with the pore structure in water maintained, water contained in the particles was replaced with ethanol and with t-butyl alcohol and freeze-dried for sample preparation. To make sure, absence of marked shrinkage and expansion in cross-linked polymer particles, associated with the sample preparation, was confirmed by digital fine scope. In the SEM observation of the surface and cross-section of the particles after distillation of gold, the presence of many minute pores of micro size or smaller was confirmed on the whole cross-linked polymer particles obtained.

(Classification)

Large particles on a sieve of 710 $\mu$m screen size and small particles under a sieve of 300 $\mu$m screen size were removed from the obtained cross-linked particles, and the following evaluations were conducted. There were a very small amount of particles on a sieve of 710 $\mu$m screen size or under a sieve of 300 $\mu$m screen size.

(Analysis of nitrogen content)

After sufficient vacuum drying of said cross-linked polymer particles, element analysis (CHN) was conducted using micro-recorder JM 10 type (J-SCIENCE-LAB Co., Ltd.) to calculate the nitrogen (N) content in dry weight at 7.8 weight %. In addition, TAIC content against all polymerization units constituting said polymer particles was calculated from the value of said element analysis as 46.1 weight % using the following formula:

$$\text{TAIC content} = \text{N content} \times \text{Molecular weight of TAIC} / (\text{Atomic weight of N} \times \text{Number of N atoms in TAIC})$$

(Measurement of volume elasticity)

The volume elasticity of said cross-linked polymer particles was measured by the following method. First, suspension of said particles, dispersed in water, was added to a 15 mL graduated hard glass cylinder of 15 mm internal diameter, and the particles were packed uniformly by tapping, etc. and particles are measured and collected correctly to make the sedimentation volume without loading to be 4 mL. Subsequently, using a piston with a diameter that is smaller than the internal diameter of the cylinder and allows the passage of water, but the passage of particles, between the pistol and cylinder, the packed particles were pressurized by autograph (manufactured by Shimadzu Corp., EZ-TEST) at a rate of 5 mm/min. Pressurization was continued until the load exceeded 20 N or packed volume reduced by 20 %, and displacement and load change were measured. The volume elasticity of said cross-linked polymer particles, when packed in the cylinder and pressurized by the piston in water, was 0.204 MPa according to the following formula:

$$\text{Volume elasticity} = (\text{Change in loading} / \text{Cross-section of cylinder}) / \text{Change of packed volume per unit volume}$$

(Measurement of activation concentration by batch contact)

1 mL of said cross-linked polymer particles, uniformly spun down in water, was measured and collected, and after replacement of water content by saline, contacted with 10 mL of heparin-added fresh blood of a healthy individual in a 37°C incubator for 30 minutes with gentle stirring. After predetermined treatment of sampled blood, activated complements (C5a, C3a) and granulocyte elastase (PMN-E) levels were measured. Even after contact with cross-linked polymer particles, the C5a and C3a levels remained relatively low, and complement system was slightly activated. Also, PMN-E level remained relatively low, and leukocytes were slightly activated.

(Evaluation of nonspecific adsorption by batch contact)

1 mL of said cross-linked polymer particles, uniformly spun down in water, was measured and collected, and after replacement of water content by saline, contacted with 6 mL of citrate-anticoagulated fresh blood of a healthy individual in a 37°C incubator for 20 minutes with gentle stirring. For the sampled plasma, fibrinogen and albumin levels were measured and compared with saline alone of the same volume containing no particle. As a result, there was little change in the fibrinogen and albumin levels, and little nonspecific adsorption to said cross-linked polymer particles.

(Measurement of thrombin-antithrombin III complex (TAT) level)

1 mL of said cross-linked polymer particles, uniformly spun down in water, was measured and collected, and after replacement of water content by heparin-added saline, contacted with 6 mL of fresh blood of a healthy individual, supplemented with a small quantity of heparin, in a 37°C incubator for 120 minutes with gentle stirring. After predetermined treatment of the sampled blood, thrombin-antithrombin III complex (TAT) level was measured. As a result, even after contact with said cross-linked polymers, the TAT level remained relatively low, and coagulation system was slightly activated.

(Column blood passage experiment)

2.7 mL of sedimentation volume of said particles, in which water content was replaced by saline, was packed into a column of 10 mm internal diameter, equipped with mesh of 150 $\mu$m screen size at the inlet and outlet, and 8.1 mL of heparin-added saline was passed to carry out priming procedure.
40 mL of heparin-added fresh blood of a healthy individual was placed in a water bath at 37°C to be used as blood pool, and the column was circulated with blood at a rate of 2.4 mL/min for 120 minutes. During that time, there was neither column clogging nor decreased flow volume. In addition, neither visible deformation nor consolidation of said polymer particles packed into the column was observed. A small quantity of blood was sampled from the inlet and outlet of the column, and blood cells were counted using a blood cell counter. As a result, passage of erythrocytes, leukocytes and platelets was all favorable, and blood cells slightly adhered to said cross-linked polymer particles. In addition, PMN-E level after 120-minute circulation was measured. As a result, the PMN-E level was relatively low, and leukocytes were slightly activated. Furthermore, CD62p positive rate after 120-minute circulation was measured as a marker that indicates platelet activation using Becton Dickinson FACS Calibur. As a result, the CD62p positive rate was relatively low, and platelets were slightly activated. No clear blood clot was observed in a column after blood passage.

(Shaking test)

5 mL of sedimentation volume of said cross-linked polymer particles, from which fine particles were preliminarily removed from supernatant by decantation, was collected and added into a glass stoppered test tube together with water to achieve 20 mL of total volume. After shaking this vigorously at 300-time to-and-fro motions/min, 10 mL of supernatant was collected, and 10 mL of water was added instead. This procedure was repeated 60 times, and the change in the number of fine particles of at least 10 $\mu$m, contained in the supernatant, was measured by Coulter counter (100 $\mu$m of aperture diameter). The particle size, measured by Coulter counter, was adjusted for volume-weighted mean diameter in water, calculated from the image taken by digital fine scope. As a result, the number of fine particles in the supernatant, after repeating vigorous shaking 60 times, was small, demonstrating that said cross-linked polymer particles hardly generated fine particles even by application of strong mechanical stimuli.

TABLE 1

| Ex. | Feed of vinyl acetate (phr) | Feed of TAIC (phr) | Feed of ethyl acetate (phr) | Feed of heptane (phr) | Feed of PVAc (phr) / Average degree of polymerization | Feed of V-65 (phr) | Feed of AIBN (phr) | Polymerization time (Hr) |
|---|---|---|---|---|---|---|---|---|
| 1 | 100 | 26.0 | 122.0 | 51.0 | 12.8/n = 400 | 5.1 | 0 | 5 |
| 2 | 100 | 24.0 | 108.0 | 49.0 | 9.6/n = 400 | 5.0 | 0 | 5 |
| 3 | 100 | 28.0 | 112.0 | 51.0 | 9.6/n = 400 | 5.2 | 0 | 5 |
| 4 | 100 | 32.0 | 116.0 | 53.0 | 9.6/n = 400 | 5.0 | 0 | 5 |
| 5 | 100 | 20.7 | 192.0 | 64.0 | 9.6/n = 800 | 4.9 | 0 | 5 |
| 7 | 100 | 24.0 | 131.5 | 48.2 | 12.8/n = 800 | 5.0 | 0 | 5 |
| 8 | 100 | 24.0 | 108.0 | 49.0 | 9.6/n = 400 | 5.0 | 0 | 5 |
| 9 | 100 | 26.0 | 122.0 | 51.0 | 12.8/n = 400 | 5.1 | 0 | 5 |
| 10 | 100 | 20.7 | 192.0 | 64.0 | 12.8/n = 500 | 4.9 | 0 | 5 |
| 11 | 100 | 31.1 | 208.5 | 69.5 | 12.8/n = 500 | 5.3 | 0 | 5 |
| 12 | 100 | 24.0 | 120.0 | 50.0 | 12.8/n = 400 | 5.0 | 0 | 5 |
| 13 | 100 | 20.7 | 128.0 | 42.7 | 12.8/n = 500 | 4.9 | 0 | 5 |
| 14 | 100 | 25.9 | 173.6 | 57.9 | 13.4/n = 500 | 2.6 | 1.7 | 8 |
| 15 | 100 | 41.4 | 150.0 | 50.0 | 7.5/n = 500 | 5.8 | 0 | 5 |

TABLE 2

| Ex. | Polymerization conversion rate of vinyl acetate (%) | Bulk specific gravity (g/mL) | Number-average particle size ($\mu$m) | Volume-weighted mean diameter ($\mu$m) | Particles of 0.8-1.2-fold volume-weighted mean diameter (volume %) | Particles of 0.9-1.1-fold volume-weighted mean diameter (volume %) | Particles of less than 100 $\mu$m (volume %) |
|---|---|---|---|---|---|---|---|
| 1 | 51 | 0.134 | 459 | 469 | 99.3 | 98.0 | 0.006 |
| 2 | 58 | 0.155 | 442 | 462 | 97.3 | 95.4 | 0.000 |
| 3 | 47 | 0.145 | 470 | 479 | 96.3 | 95.7 | 0.000 |
| 4 | 48 | 0.140 | 468 | 485 | 93.2 | 91.1 | 0.000 |
| 5 | 41 | 0.104 | 408 | 486 | 93.0 | 86.2 | 0.042 |
| 6 | - | 0.483 | 404 | 409 | 98.8 | 95.3 | 0.000 |
| 7 | 54 | 0.142 | 332 | 472 | 74.0 | 49.1 | 0.077 |
| 8 | 60 | 0.156 | 308 | 480 | 66.8 | 44.8 | 0.131 |
| 9 | 46 | 0.138 | 383 | 486 | 68.1 | 35.8 | 0.020 |
| 10 | 36 | 0.096 | 301 | 421 | 64.9 | 35.8 | 0.138 |
| 11 | 35 | 0.080 | 286 | 438 | 72.0 | 41.4 | 0.141 |
| 12 | 60 | 0.144 | 323 | 468 | 63.4 | 34.1 | 0.055 |
| 13 | 55 | 0.150 | 330 | 510 | 68.9 | 35.0 | 0.086 |
| 14 | 91 | 0.122 | 238 | 412 | 56.3 | 33.1 | 0.396 |
| 15 | 29 | 0.127 | 315 | 433 | 69.7 | 41.4 | 0.064 |

(continued)

| Ex. | Polymerization conversion rate of vinyl acetate (%) | Bulk specific gravity (g/mL) | Number-average particle size (μm) | Volume-weighted mean diameter (μm) | Particles of 0.8-1.2-fold volume-weighted mean diameter (volume %) | Particles of 0.9-1.1-fold volume-weighted mean diameter (volume %) | Particles of less than 100 μm (volume %) |
|---|---|---|---|---|---|---|---|
| 16 | - | 0.299 | 369 | 500 | 64.5 | 38.5 | 0.034 |
| 17 | - | 0.055 | 457 | 485 | 87.2 | 46.4 | 0.013 |

EXAMPLE 2

436.9 g of water, 1.81 g of 3 weight % aqueous solution of sodium α-olefin sulphonate and 124.2 g of 10 weight % slurry of particulate tribasic calcium phosphate were fed into a 2 L separable flask with a stirring blade and gently mixed.

An instrument, in which a nozzle to spray monomer mixture to a column filled with dispersion medium is inserted, the upper part of the nozzle is made of an orifice plate with at least 12 small openings of 0.17 mm pore size, an oscillation plate to transmit oscillation is set at the lower part, and the oscillation plate is connected to oscillation exciter, was used as liquid drop generator. A tube to supply dispersion medium from a dispersion medium tank was connected to a column, and a tube to supply monomer mixture from a monomer mixture tank was connected to the nozzle. A double plunger pump with high quantitative capability and less pulsing was used for the supply of monomer mixture.

517.4 g of monomer mixture of the composition shown in Table 1 was supplied from a monomer mixture tank to the nozzle at a rate of 27.6 mL/min and fed into a separable flask via liquid drop generator. Simultaneously, 560.8 g of the same dispersion medium as in Example 1 was supplied from the dispersion medium tank to the column. The monomer mixture was sent into the separable flask for polymerization in the same manner as in Example 1, except for the application of mechanical oscillation of 0.2 G strength to the monomer mixture; the polymerization conversion rate of vinyl acetate was 58 %. Aftertreatment was conducted in the same manner as in Example 1, and particle size and bulk specific gravity were measured. The results are shown in Table 2. Samples for SEM observation were prepared in the same manner as in Example 1, and the surface and cross-section were observed by SEM to find the presence of many minute pores of micro size or smaller on the whole cross-linked polymer particles obtained. Classification was conducted in the same manner as in Example 1, and the following evaluations were made. There were a small amount of particles that were regard as classification loss.

Nitrogen content was analyzed in the same manner as in Example 1; N content of said polymer particle in dry weight was 7.1 weight %. The percentage of TAIC unit against total polymerization units that constitute said cross-linked polymer particle was 41.9 weight %.

(Analysis of surface nitrogen content: X-ray photoelectron spectrometry)

After sufficient further drying of sample that was prepared by t-butyl alcohol vacuum freeze-drying for said SEM observation, X-ray photoelectron spectrometry (XPS) of the surface of said particle was conducted using Quantum 2000 [ULVAC-PHI, INC.; X-ray strength: AlKα/15 kV·25 W, X-ray beam diameter: 100 μm diameter, pass energy: 187.85 eV (wide spectrum) 58.70 eV (narrow spectrum)] as an instrument. The surface nitrogen (N) concentration (atomic percent: at%) was calculated as 6.1 at%. Based upon said surface nitrogen (N) concentration, percentage of triallyl isocyanurate (TAIC) unit on the surface of said cross-linked polymer particle was calculated according to the following formula (1), and converted to weight % according to the formula (2) as 35.3 weight %.

$$\text{Surface TAIC} = 1 - \{(1 - 6 \times \text{Surface N}) / (1 - 5 \times \text{Surface N})\} \quad \text{Formula (1)}$$

$$\text{Surface TAIC (Weight)} = 249.27 \times \text{Surface TAIC} / \{249.27 \times \text{Surface TAIC} + 44.05 \times (1 - \text{Surface TAIC})\} \quad \text{Formula (2)}$$

The cross-linked polymer particles were contacted with fresh blood of a healthy individual in the same manner as in Example 1, and activated complements (C5a, C3a) and granulocyte elastase (PMN-E) levels were measured. The C5a and C3a levels remained relatively low, and complement system was slightly activated. Also, PMN-E level remained relatively low, and leukocytes were slightly activated.

Column blood passage experiment was conducted in the same manner as in Example 1. There was neither column clogging nor decreased flow volume. In addition, passage of erythrocytes, leukocytes and platelets was all favorable, and blood cells slightly adhered to said cross-linked polymer particles. In addition, CD62p positive rate was relatively low, and platelets were slightly activated. No clear blood clot was observed in a column after blood passage.

EXAMPLE 3

579.7 g of water, 1.87 g of 3 weight % aqueous solution of sodium $\alpha$-olefin sulphonate and 128.8 g of 10 weight % slurry of particulate tribasic calcium phosphate were fed into a 2 L separable flask with a stirring blade and gently mixed.

534.9 g of monomer mixture of the composition shown in Table 1 was supplied to a nozzle at a rate of 27.6 mL/min in the same manner as in Example 1. Simultaneously, 582.2 g of the same dispersion medium as in Example 1 was supplied from a dispersion medium tank to the column. The monomer mixture was sent into the separable flask for polymerization in the same manner as in Example 1, except for the application of mechanical oscillation of 0.2 G strength to the monomer mixture; the polymerization conversion rate of vinyl acetate was 47 %. Aftertreatment was conducted in the same manner as in Example 1, and particle size and bulk specific gravity were measured. The results are shown in Table 2. SEM observation was conducted in the same manner as in Example 1 to find the presence of many minute pores of micro size or smaller on the whole cross-linked polymer particles obtained. Classification was conducted in the same manner as in Example 1, and the following evaluations were conducted. There were a small amount of particles that were regard as classification loss. Nitrogen content was analyzed in the same manner as in Example 1; N content of said polymer particle in dry weight was 7.9 weight %. The percentage of TAIC unit against total polymerization units that constitute said cross-linked polymer particle was 46.5 weight %. Volume elasticity was 0.313 MPa.

The cross-linked polymer particles were contacted with fresh blood and plasma of a healthy individual in the same manner as in Example 1, and activated complements (C5a, C3a), granulocyte elastase (PMN-E), fibrinogen and albumin levels were measured. The C5a and C3a levels remained relatively low, and complement system was slightly activated. Also, PMN-E level remained relatively low, and leukocytes were slightly activated. There was little change in the fibrinogen and albumin levels, and little nonspecific adsorption to said cross-linked polymer particles.

Column blood passage experiment was conducted in the same manner as in Example 1. There was neither column clogging nor decreased flow volume. In addition, passage of erythrocytes, leukocytes and platelets was all favorable, and blood cells slightly adhered to said cross-linked polymer particles. No clear blood clot, etc. was observed in a column after blood passage.

Shaking test was conducted in the same manner as in Example 1, and it was confirmed that said cross-linked polymer particles hardly generated fine particles even by application of strong mechanical stimuli.

EXAMPLE 4

1,776.3 g of water, 7.4 g of 3 weight % aqueous solution of sodium $\alpha$-olefin sulphonate and 507.5 g of 10 weight % slurry of particulate tribasic calcium phosphate were fed into an 8 L separable flask with a stirring blade and gently mixed.

2,114.2 g of monomer mixture of the composition shown in Table 1 was supplied to nozzle at a rate of 27.6 mL/min in the same manner as in Example 1. Simultaneously, 2,297.4 g of the same dispersion medium as in Example 1 was supplied from a dispersion medium tank to the column.

The monomer mixture was sent into the separable flask for polymerization in the same manner as in Example 1, except for the application of mechanical oscillation of 0.2 G strength to the monomer mixture; the polymerization conversion rate of vinyl acetate was 48 %. Aftertreatment was conducted in the same manner as in Example 1, and particle size and bulk specific gravity were measured. The results are shown in Table 2. SEM observation was conducted in the same manner as in Example 1 to find the presence of many minute pores of micro size or smaller on the whole particle. Classification was conducted in the same manner as in Example 1, and the following evaluations were conducted. There were a small amount of particles that were regard as classification loss.

Nitrogen content was analyzed in the same manner as in Example 1; N content of said polymer particle in dry weight was 8.4 weight %. The percentage of TAIC unit against total polymerization units that constitute said cross-linked polymer particle was 49.6 weight %. Volume elasticity was 0.325 MPa.

The cross-linked polymer particles were contacted with fresh blood and plasma of a healthy individual in the same manner as in Example 1, and activated complements (C5a, C3a), granulocyte elastase (PMN-E), fibrinogen and albumin levels were measured. The C5a and C3a levels remained relatively low, and complement system was slightly activated. Also, PMN-E level remained relatively low, and leukocytes were slightly activated. There was little change in the fibrinogen and albumin levels, and little nonspecific adsorption to said cross-linked polymer particles.

Column blood passage experiment was conducted in the same manner as in Example 1. There was neither column clogging nor decreased flow volume. In addition, passage of erythrocytes, leukocytes and platelets was all favorable, and blood cells slightly adhered to said cross-linked polymer particles. No clear blood clot was observed in a column after blood passage.

Shaking test was conducted in the same manner as in Example 1, and it was confirmed that said cross-linked polymer particles hardly generated fine particles even by application of strong mechanical stimuli.

EXAMPLE 5

16.05 g of water, 74.2 g of 3 weight % aqueous solution of sodium $\alpha$-olefin sulphonate and 5.09 g of 10 weight % slurry of particulate tribasic calcium phosphate were fed into a 100 L polymerization reactor with jacket and stirring blade and gently mixed.

An instrument, in which three nozzles to spray monomer mixture to a column filled with dispersion medium is inserted, the upper part of the nozzle is made of an orifice plate with at least 45 small openings of 0.17 mm pore size, an oscillation plate to transmit oscillation is set at the lower part, and the oscillation plate is connected to oscillation exciter, was used as liquid drop generator. A tube to supply dispersion medium from dispersion medium tank was connected to a column, and a tube to supply monomer mixture from a monomer mixture tank was connected to the nozzle. A triple plunger pump with high quantitative capability and less pulsing was used for the supply of monomer mixture. Said liquid drop generator was connected to the bottom of polymerization reactor via a liquid drop tube.

19.53 kg of monomer mixture of the composition shown in Table 1 was supplied to the nozzle at a rate of 338 mL/min and fed into polymerization reactor via liquid drop generator. Simultaneously, 21.21 kg of aqueous solution, containing 0.3 weight % PVA and 120 ppm sodium nitrite, was supplied as dispersion medium from dispersion medium tank to the column at a rate of 287 mL/min and fed into the polymerization reactor via the liquid drop generator.

At this time, mechanical oscillation at 400 Hz was provided to the monomer mixture by oscillation exciter. The monomer mixture, spurted out from the nozzle opening, was divided by said mechanical oscillation to form uniformly-sized liquid drop group in the column, and the formed liquid drops were sent into polymerization reactor with dispersion medium that was supplied simultaneously.

After introduction of the liquid drop group, generated in said liquid drop generator, into polymerization reactor, nitrogen replacement was carried out in the polymerization reactor, and the internal temperature was maintained at 65°C for 5 hours for polymerization. The polymerization conversion rate of vinyl acetate was 41 %.

Aftertreatment was conducted in the same manner as in Example 1, and particle size and bulk specific gravity were measured. The results are shown in Table 2. SEM observation was conducted in the same manner as in Example 1 to find the presence of many minute pores of micro size or smaller on the whole particle. Classification was conducted in the same manner as in Example 1, and the following evaluations were conducted. There were a small amount of particles that were regard as classification loss.

Nitrogen content was analyzed in the same manner as in Example 1; N content of said cross-linked polymer particles in dry weight was 8.1 weight %. The percentage of TAIC unit against total polymerization units that constitute said cross-linked polymer particle was 48.0 weight %. Volume elasticity was 0.034 MPa.

The cross-linked polymer particles were contacted with fresh blood and plasma of a healthy individual in the same manner as in Example 1, and activated complements (C5a, C3a), granulocyte elastase (PMN-E), fibrinogen, albumin and thrombin-antithrombin III complex (TAT) levels were measured. The C5a and C3a levels remained relatively low, and complement system was slightly activated. Also, PMN-E level remained relatively low, and leukocytes were slightly activated. There was little change in the fibrinogen and albumin levels, and little nonspecific adsorption to said cross-linked polymer particles. The TAT level remained relatively low, and coagulation system was slightly activated.

Column blood passage experiment was conducted in the same manner as in Example 1. There was neither column clogging nor decreased flow volume. In addition, passage of erythrocytes, leukocytes and platelets was all favorable, and blood cells slightly adhered to said polymer particles. As a result of measurement of PMN-E level after 120-minute circulation, the PMN-E level was relatively low, and leukocytes were slightly activated. In addition, after 120-minute circulation, CD62p positive rate was relatively low, and platelets were slightly activated. No clear blood clot was observed in a column after blood passage.

Shaking test was conducted in the same manner as in Example 1, and it was confirmed that said cross-linked polymer particles hardly generated fine particles even by application of strong mechanical stimuli.

EXAMPLE 6

521 g of water was fed into a 2 L separable flask with stirring blade and gently mixed.

The same liquid drop generator, except that orifice plate with 6 small openings of 0.17 mm pore size, was used, and 504 g monomer mixture, consisting of 70 parts by weight of methylmethacrylate (MMA), 30 parts by weight of ethylene glycol dimethacrylate (EGDMA), 52.5 parts by weight of ethyl acetate, 7.5 parts by weight of heptane and 1.0 parts by weight of V-65, was fed into the separable flask via liquid drop generator. Simultaneously, 464 g of dispersion medium, consisting of 120.7 g of 3 weight % of PVA aqueous solution, 1.81 g of 6 weight % of sodium nitrite aqueous solution and 1,877 g of water, was supplied to a column and fed into the separable flask via liquid drop generator.

At this time, mechanical oscillation at constant frequency (400 Hz) and intensity of 1.3 G was provided to monomer mixture by oscillation exciter. The liquid column of monomer mixture, spurted out from the nozzle opening, was divided by said mechanical oscillation to form uniformly-sized liquid drops in dispersion medium in the column. The formed liquid drops were sent into the separable flask together with dispersion medium that was supplied simultaneously. Subsequently, said liquid drops were polymerized in the same manner as in Example 1.

Said liquid drops were sampled at each time point of column outlet, before polymerization and after polymerization, and were observed in the same manner as in Example 1. All of said liquid drops maintained uniform size, demonstrating that polymerization was carried out without connation and additional dispersion.

After the first polymerization, 41 g of 2-hydroxyethyl methacrylate (HEMA) and 205 g of 10 weight % of sodium persulfate aqueous solution were added to the content of the separable flask and maintained at 60°C for 4 hours for graft polymerization of 2-hydroxyethyl methacrylate to the polymer particles generated by the first polymerization.

After the second polymerization, the obtained polymerized particles were thoroughly washed with water, subsequently with acetone, and additionally with methanol. After replacement of the methanol by water, washing was conducted thoroughly with hot water at 80°C. Subsequently, autoclaving was conducted at 121°C for 20 minutes to obtain clean polymer particles.

Said polymer particles were measured and observed in the same manner as in Example 1. The number-averaged particle size and volume-weighted mean diameter of the obtained polymer particles in water were 404 $\mu$m and 409 $\mu$m, respectively. Particles of 0.8 to 1.2-fold volume-weighted mean diameter accounted for 98.8 volume %, particles of 0.9 to 1.1-fold volume-weighted mean diameter accounted for 95.3 volume %, and particles of less than 100 $\mu$m could not be observed by 3D digital fine scope. Bulk specific gravity was 0.483 g/mL. In the SEM observation of the surface and cross-section, the presence of many minute pores was confirmed on the whole particle.

Large particles on a sieve of 710 $\mu$m screen size and small particles and fine particles under a sieve of 300 $\mu$m screen size were removed from the obtained cross-linked particles, and the following evaluations were conducted. There were a very small amount of particles on a sieve of 710 $\mu$m screen size or under a sieve of 300 $\mu$m screen size.

Blood circulation in a column packed with said polymer particles in the same manner as in Example 1 was evaluated. As a result, there was neither column clogging nor decreased flow volume during the blood circulation. In addition, passage of erythrocytes, leukocytes and platelets was all favorable, and blood cells slightly adhered to said polymer particles. In addition, after 120-minute circulation, CD62p positive rate was relatively low, and platelets were slightly activated. No clear blood clot, etc. was observed in a column after blood passage.

EXAMPLE 7

501.6 g of monomer mixture of the composition shown in Table 1 was added to a 2 L separable flask with plate-like stirring blade and two baffle plate, preliminarily fed with 1,045.2 g of aqueous phase containing 1,000 parts by weight of water, 0.15 parts by weight of polyvinyl alcohol, 0.033 parts by weight of $\alpha$-olefin sodium sulphonate, 7.45 parts by weight of particulate tribasic calcium phosphate (solid content) and 0.056 parts by weight of sodium nitrite, at room temperature. After sufficient shaking/mixing and nitrogen replacement, suspension polymerization was carried out while the internal temperature was maintained at 65°C for 5 hours; the polymerization conversion rate of vinyl acetate was 54 %.

Aftertreatment was conducted in the same manner as in Example 1, and particle size and bulk specific gravity were measured. The results are shown in Table 2.

SEM observation was conducted in the same manner as in Example 1 to find the presence of many minute pores of micro size or smaller on the whole particle.

Classification was conducted in the same manner as in Example 1, and the following evaluations were conducted.

Nitrogen content was analyzed in the same manner as in Example 1; N content of said polymer particle in dry weight was 7.8 weight %. The percentage of TAIC unit against total polymerization units that constitute said cross-linked polymer particle was 46.3 weight %. Volume elasticity was 0.200 MPa.

XPS analysis was conducted in the same manner as in Example 2 to calculate surface N concentration as 7.4 at%. In addition, the percentage of TAIC unit on the surface of said cross-linked polymer particles was calculated as 43.0 weight %.

The cross-linked polymer particles were contacted with fresh blood and plasma of a healthy individual in the same manner as in Example 1, and activated complements (C5a, C3a), granulocyte elastase (PMN-E), fibrinogen, albumin and thrombin-antithrombin III complex (TAT) levels were measured. The C5a and C3a levels remained relatively low, and complement system was slightly activated. Also, PMN-E level remained relatively low, and leukocytes were slightly activated. There was little change in the fibrinogen and albumin levels, and little nonspecific adsorption to said cross-linked polymer particles. The TAT level remained relatively low, and coagulation system was slightly activated.

Column blood passage experiment was conducted in the same manner as in Example 1. There was neither column clogging nor decreased flow volume. In addition, passage of erythrocytes, leukocytes and platelets was all favorable, and blood cells slightly adhered to said polymer particles. As a result of measurement of PMN-E level after 120-minute circulation, the PMN-E level was relatively low, and leukocytes were slightly activated. No clear blood clot, etc. was observed in a column after blood passage.

Shaking test was conducted in the same manner as in Example 1, and it was confirmed that said cross-linked polymer particles hardly generated fine particles even by application of strong mechanical stimuli.

EXAMPLE 8

Suspension polymerization was carried out with 502.6 g of monomer mixture of the composition shown in Table 1 and 1,088.9 g of aqueous phase in the same manner as in Example 7. The polymerization conversion rate of vinyl acetate was 60 %. Aftertreatment was conducted in the same manner as in Example 1, and particle size and bulk specific gravity were measured. The results are shown in Table 2. SEM observation was conducted in the same manner as in Example 1 to find the presence of many minute pores of micro size or smaller on the whole particle. Classification was conducted in the same manner as in Example 1, and the following evaluations were conducted.

Nitrogen content was analyzed in the same manner as in Example 1; N content of said polymer particle in dry weight was 7.3 weight %. The percentage of TAIC unit against total polymerization units that constitute said cross-linked polymer particle was 43.2 weight %. Volume elasticity was 0.222 MPa.

The cross-linked polymer particles were contacted with fresh blood and plasma of a healthy individual in the same manner as in Example 1, and activated complements (C5a, C3a), fibrinogen, albumin and thrombin-antithrombin III complex (TAT) levels were measured. The C5a and C3a levels remained relatively low, and complement system was slightly activated. Also, PMN-E level remained relatively low, and leukocytes were slightly activated. There was little change in the fibrinogen and albumin levels, and little nonspecific adsorption to said cross-linked polymer particles. The TAT level remained relatively low, and coagulation system was slightly activated.

Shaking test was conducted in the same manner as in Example 1, and it was confirmed that said cross-linked polymer particles hardly generated fine particles even by application of strong mechanical stimuli.

EXAMPLE 9

Suspension polymerization was carried out with 522.9 g of monomer mixture of the composition shown in Table 1 and 1,132.9 g of aqueous phase in the same manner as in Example 7. The polymerization conversion rate of vinyl acetate was 47 %. Aftertreatment was conducted in the same manner as in Example 1, and particle size and bulk specific gravity were measured. The results are shown in Table 2. SEM observation was conducted in the same manner as in Example 1 to find the presence of many minute pores of micro size or smaller on the whole particle. Classification was conducted in the same manner as in Example 1, and the following evaluations were conducted.

Nitrogen content was analyzed in the same manner as in Example 1; N content of said polymer particle in dry weight was 8.3 weight %. The percentage of TAIC unit against total polymerization units that constitute said cross-linked polymer particle was 49.0 weight %. Volume elasticity was 0.239 MPa.

The cross-linked polymer particles were contacted with fresh blood and plasma of a healthy individual in the same manner as in Example 1, and activated complements (C5a, C3a), granulocyte elastase (PMN-E), fibrinogen and albumin levels were measured. The C5a and C3a levels remained relatively low, and complement system was slightly activated. Also, PMN-E level remained relatively low, and leukocytes were slightly activated. There was little change in the fibrinogen and albumin levels, and little nonspecific adsorption to said cross-linked polymer particles.

Shaking test was conducted in the same manner as in Example 1, and it was confirmed that said cross-linked polymer particles hardly generated fine particles even by application of strong mechanical stimuli.

EXAMPLE 10

Suspension polymerization was carried out with 512.8 g of monomer mixture of the composition shown in Table 1 and 1,114.1 g of aqueous phase in the same manner as in Example 7. The polymerization conversion rate of vinyl acetate was 36 %. Aftertreatment was conducted in the same manner as in Example 1, and particle size and bulk specific gravity were measured. The results are shown in Table 2. SEM observation was conducted in the same manner as in Example 1 to find the presence of many minute pores of micro size or smaller on the whole particle. Classification was conducted in the same manner as in Example 1, and the following evaluations were conducted.

Nitrogen content was analyzed in the same manner as in Example 1; N content of said polymer particle in dry weight was 7.6 weight %. The percentage of TAIC unit against total polymerization units that constitute said cross-linked polymer particle was 45.2 weight %. Volume elasticity was 0.045 MPa.

The cross-linked polymer particles were contacted with fresh blood and plasma of a healthy individual in the same manner as in Example 1, and activated complements (C5a, C3a), fibrinogen and albumin levels were measured. The C5a and C3a levels remained relatively low, and complement system was slightly activated. There was little change in the fibrinogen and albumin levels, and little nonspecific adsorption to said cross-linked polymer particles.

Blood passage experiment was conducted in the same manner as in Example 1. There was neither column clogging nor decreased flow volume. In addition, passage of erythrocytes, leukocytes and platelets was all favorable, and blood cells slightly adhered to said polymer particles. No clear blood clot was observed in a column after blood passage.

Shaking test was conducted in the same manner as in Example 1, and it was confirmed that said cross-linked polymer particles hardly generated fine particles even by application of strong mechanical stimuli.

EXAMPLE 11

Suspension polymerization was carried out with 534 g of monomer mixture of the composition shown in Table 1 and 1,160.1 g of aqueous phase in the same manner as in Example 7. The polymerization conversion rate of vinyl acetate was 35 %. Aftertreatment was conducted in the same manner as in Example 1, and particle size and bulk specific gravity were measured. The results are shown in Table 2. The bulk specific gravity was relatively low: 0.080 g/mL. SEM observation was conducted in the same manner as in Example 1 to find the presence of many minute pores of micro size or smaller on the whole particle. Classification was conducted in the same manner as in Example 1, and the following evaluations were conducted.

Nitrogen content was analyzed in the same manner as in Example 1; N content of said polymer particle in dry weight was 9.3 weight %. The percentage of TAIC unit against total polymerization units that constitute said cross-linked polymer particle was 55.1 weight %. Volume elasticity was 0.050 MPa. XPS analysis was conducted in the same manner as in Example 2 to calculate surface N concentration as 9.9 at%. In addition, the percentage of TAIC unit on the surface of said cross-linked polymer particles was calculated as 58.0 weight %.

The cross-linked polymer particles were contacted with fresh blood and plasma of a healthy individual in the same manner as in Example 1, and activated complements (C5a, C3a), fibrinogen, albumin and thrombin-antithrombin III complex (TAT) levels were measured. The C5a and C3a levels remained relatively low, and complement system was slightly activated. Also, PMN-E level remained relatively low, and leukocytes were slightly activated. There was little change in the fibrinogen and albumin levels, and little nonspecific adsorption to said cross-linked polymer particles. The TAT level remained relatively low, and coagulation system was slightly activated. Shaking test was conducted in the same manner as in Example 1, and it was confirmed that said cross-linked polymer particles somewhat tended to generate fine particles by mechanical stimuli.

EXAMPLE 12

Suspension polymerization was carried out in an 8 L separable flask (having plate-like stirring blade and two baffle plates) with 2,245.3 g of monomer mixture of the composition shown in Table 1 and 4,864.4 g of aqueous phase in the same manner as in Example 7. The polymerization conversion rate of vinyl acetate was 60 %. Aftertreatment was conducted in the same manner as in Example 1, and particle size and bulk specific gravity were measured. The results are shown in Table 2. SEM observation was conducted in the same manner as in Example 1 to find the presence of many minute pores of micro size or smaller on the whole particle. Classification was conducted in the same manner as in Example 1, and the following evaluations were conducted.

Nitrogen content was analyzed in the same manner as in Example 1; N content of said polymer particle in dry weight was calculated as 7.0 weight %. The percentage of TAIC unit against total polymerization units that constitute said cross-linked polymer particle was 41.6 weight %.

XPS analysis was conducted in the same manner as in Example 2 to calculate surface N concentration as 5.8 at%. In addition, the percentage of TAIC unit on the surface of said cross-linked polymer particle was calculated as 33.5 weight %.

The cross-linked polymer particles were contacted with fresh blood and plasma of a healthy individual in the same manner as in Example 1, and activated complements (C5a, C3a) levels were measured. The C5a and C3a levels remained relatively low, and complement system was slightly activated.

EXAMPLE 13

Suspension polymerization was carried out with 525.5 g of monomer mixture of the composition shown in Table 1 and 1,141.6 g of aqueous phase in the same manner as in Example 7. The polymerization conversion rate of vinyl acetate was 55 %. Aftertreatment was conducted in the same manner as in Example 1, and particle size and bulk specific gravity were measured. The results are shown in Table 2. SEM observation was conducted in the same manner as in Example 1 to find the presence of many minute pores of micro size or smaller on the whole particle. Classification was conducted in the same manner as in Example 1, and there were large amounts of polymer particles on a sieve of 710 $\mu$m screen size or under a sieve of 300 $\mu$m screen size, demonstrating marked classification loss. The number-averaged particle size and volume-weighted mean diameter after classification were 469 $\mu$m and 513 $\mu$m, respectively. Polymer particles of 0.8 to 1.2-fold volume-weighted mean diameter after classification accounted for 80.9 volume %, however, polymer particles of 0.9 to 1.1-fold volume-weighted mean diameter accounted for only 53.2 volume %.

Nitrogen content was analyzed in the same manner as in Example 1; N content of said polymer particle in dry weight was 6.8 weight %. The percentage of TAIC unit against total polymerization units that constitute said cross-linked polymer particle was 40.5 weight %. Volume elasticity was 0.12 MPa.

The cross-linked polymer particles were contacted with fresh blood and plasma of a healthy individual in the same manner as in Example 1, and activated complements (C5a, C3a), granulocyte elastase (PMN-E), fibrinogen, albumin and thrombin-antithrombin III complex (TAT) levels were measured. The C5a and C3a levels were relatively high, and complement system was activated. Also, PMN-E level was relatively high, and leukocytes were activated. The TAT level was relatively high, and coagulation system was activated. There was little change in the fibrinogen and albumin levels, and little nonspecific adsorption to said cross-linked polymer particles.

Column blood passage experiment was conducted in the same manner as in Example 1. There was neither column clogging nor decreased flow volume. In addition, passage of leukocytes and platelets was relatively low, and leukocytes and platelets adhered to said polymer particles. As a result of measurement of granulocyte elastase (PMN-E) level after 120-minute circulation, the PMN-E level was relatively high, and leukocytes were activated.

EXAMPLE 14

524.9 g of monomer mixture, consisting of 100 parts by weight of vinyl acetate, 25.9 parts by weight of TAIC, 173.6 parts by weight of ethyl acetate, 57.9 parts by weight of heptane, 13.4 parts by weight of polyvinyl acetate (PVAc) (average degree of polymerization: 500), 2.6 parts by weight of 2,2'-azobis (2,4-dimethyl valeronitrile) (V-65) and 1.7 parts by weight of 2,2'-azobis (isobutyronitrile) (AIBN), was added to a 2 L separable flask with a plate-like stirring blade and two baffle plate, preliminarily fed with 1,140.3 g of aqueous phase, containing 808.3 parts by weight of water, 0.119 parts by weight of polyvinyl alcohol, 0.027 parts by weight of $\alpha$-olefin sodium sulphonate, 6.02 parts by weight of particulate tribasic calcium phosphate (solid content) and 0.046 parts by weight of sodium nitrite, at room temperature. After sufficient shaking/mixing and nitrogen replacement, suspension polymerization was carried out while the internal temperature was maintained at 65°C for 8 hours; the polymerization conversion rate of vinyl acetate was 91 %.

Aftertreatment was conducted in the same manner as in Example 1, and particle size and bulk specific gravity were measured. The results are shown in Table 2. SEM observation was conducted in the same manner as in Example 1 to find the presence of many minute pores of micro size or smaller on the whole particle. Classification was conducted in the same manner as in Example 1, and the following evaluations were conducted.

Nitrogen content was analyzed in the same manner as in Example 1; N content of said polymer particle in dry weight was 6.9 weight %. The percentage of TAIC unit against total polymerization units that constitute said cross-linked polymer particle was 41.0 weight %. Volume elasticity was 0.096 MPa.

The cross-linked polymer particles were contacted with fresh blood and plasma of a healthy individual in the same manner as in Example 1, and activated complements (C5a, C3a), granulocyte elastase (PMN-E), fibrinogen and albumin levels were measured. The C5a and C3a levels were relatively high, and complement system was activated. Also, PMN-E level was relatively high, and leukocytes were activated. There was little change in the fibrinogen and albumin levels, and little nonspecific adsorption to said cross-linked polymer particles.

Shaking test was conducted in the same manner as in Example 1, and it was confirmed that said cross-linked polymer particles hardly generated fine particles even by application of strong mechanical stimuli.

EXAMPLE 15

Suspension polymerization was carried out with 354.7 g of monomer mixture of the composition shown in Table 1 and 1,125.1 g of aqueous phase in the same manner as in Example 7. The polymerization conversion rate of vinyl acetate was 29 %. Aftertreatment was conducted in the same manner as in Example 1, and particle size and bulk specific gravity were measured. The results are shown in Table 2. SEM observation was conducted in the same manner as in Example 1 to find the presence of many minute pores of micro size or smaller on the whole particle. Classification was conducted in the same manner as in Example 1, and there were large amounts of polymer particles on a sieve of 710 μm screen size or under a sieve of 300 μm screen size, demonstrating marked classification loss. The number-averaged particle size and volume-weighted mean diameter of polymer particles in water after classification were 435 μm and 464 μm, respectively. Polymer particles of 0.8 to 1.2-fold volume-weighted mean diameter after classification accounted for 81.2 volume %, however, particles of 0.9 to 1.1-fold volume-weighted mean diameter accounted for only 47.9 volume %.

The cross-linked polymer particles were contacted with fresh blood and plasma of a healthy individual in the same manner as in Example 1, and activated complements (C5a, C3a) and granulocyte elastase (PMN-E) levels were measured. The C5a and C3a levels remained relatively low, and complement system was slightly activated. Also, PMN-E level remained relatively low, and leukocytes were slightly activated.

EXAMPLE 16

523.7 g of monomer mixture, consisting of 82.8 parts by weight of MMA, 17.2 parts by weight of EGDMA, 128 parts by weight of ethyl acetate, 42.7 parts by weight of heptane and 4.9 parts by weight of V-65, was added to 1,178.3 g of aqueous phase, containing 595.6 parts by weight of water, 0.732 parts by weight of PVA and 0.033 parts by weight of sodium nitrite, for suspension polymerization in the same manner as in Example 1.

At this time, samples collected before and after polymerization were observed in the same manner as in Example 1; wide particle size distribution containing large and small particles was indicated at both time points.

After polymerization for the predetermined time, the content of separable flask was cooled to room temperature. Subsequently, polymerized particles were thoroughly washed with water, and with acetone. After replacement with acetone, washing was conducted with hot water at 80°C. Subsequently, autoclaving was conducted at 121°C for 20 minutes to obtain clean polymer particles. Particle size and bulk specific gravity were measured in the same manner as in Example 1. The results are shown in Table 2. SEM observation was conducted in the same manner as in Example 1 to find the presence of many minute pores of micro size or smaller on the whole particle. Classification was conducted in the same manner as in Example 1, and there were large amounts of polymer particles on a sieve of 710 μm screen size or under a sieve of 300 μm screen size, demonstrating marked classification loss. The number-averaged particle size and volume-weighted mean diameter of polymer particles in water after classification were 471 μm and 516 μm, respectively. In said polymer particles, polymer particles of 0.8 to 1.2-fold volume-weighted mean diameter after classification accounted for 83.1 volume %, however, particles of 0.9 to 1.1-fold volume-weighted mean diameter accounted for only 50.4 volume %.

Blood circulation in a column packed in the same manner as in Example 1 was evaluated. As a result, there was neither column clogging nor decreased flow volume, however, passage of leukocytes and platelets was low, and leukocytes and platelets adhered to said polymer particles. In addition, after 120-minute circulation, CD62p positive rate was relatively high, and platelets were activated.

EXAMPLE 17

Instead of cross-linked polymer particle comprising polymerization unit that contains vinyl alcohol and nitrogen, porous cellulose particle of 0.055 g/mL of bulk specific gravity was used.

Hereinafter, measurement was conducted in the same manner as in Example 1. The number-averaged particle size and volume-weighted mean diameter of said cellulose particles in water were 457 μm and 485 μm, respectively. Particles of 0.8 to 1.2-fold volume-weighted mean diameter accounted for 87.2 volume %, particles of 0.9 to 1.1-fold volume-weighted mean diameter accounted for 46.4 volume %, and particles of less than 100 μm accounted for 0.013 volume %. Volume elasticity was 0.22 MPa. In the SEM observation of the surface and cross-section of said cellulose particle, the presence of many minute pores of micro size or smaller was confirmed on the whole particle.

As a result of evaluation using blood, after contact with said cellulose particles, activated complement levels (C5a, C3a) were relatively high, and complement system was activated. In addition, after contact with said cellulose particles, granulocyte elastase (PMN-E) level was relatively high, and leukocytes were activated.

In a blood circulation experiment conducted with said cellulose particles packed into a column, neither visible deformation nor consolidation of said cellulose particles was observed, however, passage rates of leukocytes and platelets were relatively low, and leukocytes and platelets adhered to said cellulose particles. In addition, granulocyte elastase (PMN-E) and β-TG levels were relatively high after 120-minute blood circulation, demonstrating the activation of leukocytes and platelets. CD62p positive rate, after 120-minute circulation, was relatively high, demonstrating activation of platelets.

TABLE 3

| Ex. | Residual rate of albumin (%) | Batch contact C3a (ng/mL) | Batch contact C5a (ng/mL) | Batch contact PMN-E (µg/L) | Passage of leukocytes in column blood passage | Passage of platelets in column blood passage | PMN-E in column blood passage (µg/L) | CD62p positive rate in column blood passage (%) |
|---|---|---|---|---|---|---|---|---|
| 1 | 100 | 3990 | <10 | 71 | excellent | excellent | 1360 | 23 |
| 2 | 100 | 5950 | 115 | 115 | excellent | excellent | - | 29 |
| 3 | 100 | 13100 | 97 | 97 | excellent | excellent | - | - |
| 4 | 100 | 2820 | 116 | 116 | excellent | excellent | - | - |
| 5 | 100 | 4700 | 175 | 175 | excellent | excellent | 1352 | 20 |
| 6 | - | - | - | - | excellent | excellent | - | 23 |
| 13 | 100 | 15200 | 339 | 339 | inferior | inferior | 3210 | - |
| 15 | - | 11600 | 121 | 121 | - | - | - | - |
| 16 | - | - | - | - | inferior | inferior | - | 77 |
| 17 | 100 | 100 | 244 | 244 | inferior | inferior | 2690 | 38 |

TABLE 4

| Ex. | N (weight %) by CHN | TAIC (weight %) by CHN | N (at %) by surface XPS | TAIC (weight %) by surface XPS | Difference in N between CHN and surface XPS | Difference in TAIC (weight %) between CHN and surface XPS | Residual rate of albumin (%) |
|---|---|---|---|---|---|---|---|
| 2 | 7.1 | 41.9 | 6.1 | 35.3 | 1.0 | 6.6 | 100 |
| 7 | 7.8 | 46.3 | 7.4 | 43.0 | 0.4 | 3.3 | 100 |
| 11 | 9.3 | 55.1 | 9.9 | 58.0 | −0.6 | −2.9 | 100 |
| 12 | 7.0 | 41.6 | 5.8 | 33.5 | 1.2 | 8.1 | 100 |
| 14 | 6.9 | 41.0 | 4.3 | 24.7 | 2.6 | 16.3 | 100 |
| 17 | – | – | – | – | – | – | 100 |

- continued -

| Ex. | C3a (ng/mL) | C5a (ng/mL) | PMN-E (µg/L) | Passage of platelets in column blood passage | Passage of leukocytes in column blood passage | PMN-E in column blood passage (µg/L) |
|---|---|---|---|---|---|---|
| 2 | 5950 | 31 | 115 | excellent | excellent | – |
| 7 | 10700 | 56 | 113 | excellent | excellent | 1360 |
| 11 | 9800 | 13 | 94 | – | – | – |
| 12 | 17000 | 123 | – | – | – | – |
| 14 | 23400 | 326 | 410 | – | – | – |
| 17 | 33700 | 405 | 244 | inferior | inferior | 2690 |

EP 1 832 607 B1

TABLE 5

| Ex. | N content (weight %) | TAIC content (weight %) | Volume elasticity (MPa) | Residual rate of fibrinogen (%) | Residual rate of albumin (%) | C3a (ng/mL) | C5a (ng/mL) |
|---|---|---|---|---|---|---|---|
| 1 | 7.8 | 46.1 | 0.204 | 100 | 100 | 3990 | <10 |
| 3 | 7.9 | 46.5 | 0.313 | 100 | 100 | 2820 | <10 |
| 4 | 8.4 | 49.6 | 0.325 | 100 | 100 | 13100 | 59 |
| 5 | 8.1 | 48.0 | 0.034 | 100 | 100 | 4700 | 14 |
| 7 | 7.8 | 46.3 | 0.200 | 100 | 100 | 10700 | 56 |
| 8 | 7.3 | 43.2 | 0.222 | 100 | 100 | 12700 | 96 |
| 9 | 8.3 | 49.0 | 0.239 | 100 | 100 | 3400 | <10 |
| 10 | 7.6 | 45.2 | 0.045 | 100 | 100 | – | <10 |
| 11 | 9.3 | 55.1 | 0.050 | 32 | 100 | 9800 | 13 |
| 13 | 6.8 | 40.5 | 0.123 | 100 | 100 | 15200 | 151 |
| 14 | 6.9 | 41.0 | 0.096 | 100 | 100 | 23400 | 326 |
| 15 | 10.3 | 61.0 | – | – | – | 11600 | 47 |
| 17 | – | – | 0.219 | 100 | 100 | 33700 | 405 |

- continued -

| Ex. | TAT (μg/L) | PMN-E (μg/L) | Passage of platelets in column blood passage | Passage of leukocytes in column blood passage | PMN-E in column blood passage (μg/L) | Number of fine particles after 60-time shaking ○: The number of fine particles of ≥10 μm is ≤100/mL. |
|---|---|---|---|---|---|---|
| 1 | – | 71 | excellent | excellent | 1360 | ○ |
| 3 | – | 97 | excellent | excellent | – | ○ |
| 4 | – | 116 | excellent | excellent | – | ○ |
| 5 | 62 | 175 | excellent | excellent | 1352 | ○ |
| 7 | 25 | 113 | excellent | excellent | 1360 | ○ |
| 8 | 32 | – | – | – | – | ○ |
| 9 | – | 88 | – | – | – | ○ |
| 10 | – | – | excellent | excellent | – | ○ |
| 11 | 13 | 94 | – | – | – | × |
| 13 | 120 | 339 | inferior | inferior | 3210 | ○ |
| 14 | – | 410 | – | – | – | ○ |
| 15 | – | 121 | – | – | – | – |
| 17 | 19 | 244 | inferior | inferior | 2690 | ○ |

INDUSTRIAL APPLICABILITY

The invention provides cross-linked polymer particle that hardly activates complement system and leukocytes and is useful as a processing material of liquid that contains physiologically active substance and/or cells, such as body fluid, and as carrier thereof. The invention also provide cross-linked polymer particle that is less likely to activate complement system and coagulation system, causes little loss of useful substance due to nonspecific adsorption, etc., causes little activation and adhesion of cells and has excellent suitability for body fluid, etc. The invention also provides a method for manufacturing uniformly-sized polymer particle, which allows stable flow of highly viscous fluid such as body fluid and causes very little outflow of fine particles, without causing large amounts of classification loss.

**Claims**

1. A cross-linked polymer particle comprising vinyl alcohol unit and nitrogen-containing polymerization unit, wherein nitrogen content in dry weight based on the total weight of said particle, determined by element analysis, is 7.0 to 13.0 weight %, and surface nitrogen content against said particle surface, determined by X-ray photoelectron spectrometry, is 5.0 to 15.0 at%, wherein
said vinyl alcohol unit is formed through hydrolysis and/or ester exchange reaction of part or all of carboxylic vinyl ester units of cross-linked polymer that contains carboxylic vinyl ester unit obtained by polymerization of monomer mixture containing carboxylic vinyl ester and vinyl compound having triazine ring, and wherein
the polymerization conversion rate of carboxylic vinyl ester in the polymerization of monomer mixture containing carboxylic vinyl ester and vinyl compound having triazine ring is 10 to 80 %.

2. The cross-linked polymer particle of claim 1, wherein the numerical difference between the percentage of said nitrogen content and the percentage of said surface nitrogen content is less than 2.0.

3. The cross-linked polymer particle of claim 1, comprising synthetic polymer including vinyl alcohol unit and nitrogen-containing polymerization unit, wherein nitrogen content in dry weight, determined by element analysis, is 7.3 to 9.2 weight %.

4. The cross-linked polymer particle of claim 1, comprising vinyl alcohol unit and nitrogen-containing polymerization unit, wherein the percentage of nitrogen-containing polymerization unit against total polymerization units that constitute cross-linked polymer particle is 41.0 to 75.0 weight %, wherein the percentage of nitrogen-containing polymerization unit on the surface of said cross-linked polymer particles is 30.0 to 85.0 weight %.

5. The cross-linked polymer particle of Claim 4, wherein the difference between the percentage of nitrogen-containing polymerization unit against total polymerization units that constitute cross-linked polymer particle and the percentage of nitrogen-containing polymerization unit on the surface of said polymer particles is less than 15.0 weight %.

6. The cross-linked polymer particle of claim 1, comprising synthetic polymer including vinyl alcohol unit and nitrogen-containing polymerization unit, wherein the percentage of nitrogen-containing polymerization unit against total polymerization units that constitute cross-linked polymer particle is 43.0 to 54.0 weight %.

7. The cross-linked polymer particle of any of claims 1 to 6, wherein the volume-weighted mean diameter of said cross-linked polymer particles is 50 to 3,000 $\mu$m, wherein more than 80 volume % of said particles have 0.8 to 1.2-fold volume-weighted mean diameter.

8. The cross-linked polymer particle of any of claims 1 to 7, wherein particles of less than 100 $\mu$m of particle size account for 5 volume %.

9. A processing material of body fluid, comprising the cross-linked polymer of any of Claims 1 to 8.

10. A manufacturing method of a polymer particle, comprising a process (a) to form uniformly-sized liquid drops of monomer mixture comprising vinyl alcohol unit and nitrogen-containing polymerization unit in dispersion medium and a process (b) to polymerize said liquid drops under the condition that neither connation nor additional dispersion occurs, wherein volume-weighted mean diameter is 50 to 3,000 $\mu$m, and 80 volume % of said particles have 0.8 to 1.2-fold volume-weighted mean diameter, wherein nitrogen content in dry weight against the total weight of said particle, determined by element analysis, is 7.0 to 13.0 weight %, and surface nitrogen content against said particle surface, determined by X-ray photoelectron spectrometry, is 5.0 to 15.0 at%, wherein said vinyl alcohol unit is formed through hydrolysis and/or ester exchange reaction of part or all of carboxylic vinyl ester units of cross-linked polymer that contains carboxylic vinyl ester unit obtained by polymerization of monomer mixture containing carboxylic vinyl ester and vinyl compound having triazine ring, and wherein the polymerization conversion rate of carboxylic vinyl ester in the polymerization of monomer mixture containing carboxylic vinyl ester and vinyl compound having triazine ring is 10 to 80 %.

**11.** The manufacturing method of a polymer particle of claim 10, comprising the process (c) for processing and modification after said process (b).

**12.** The manufacturing method of a polymer particle of claims 10 or 11, wherein the volume-weighted mean diameter of polymer particles are 150 to 3,000 $\mu$m, wherein particles of less than 100 $\mu$m of particle size account for at most 5 volume %.

**13.** The manufacturing method of a polymer particle of any of claims 10 to 12, wherein polymer particle has at least one kind of hydrophilic group, selected from hydroxyl, polyoxyethylene and pyrrolidone.

**14.** The manufacturing method of a polymer particle of any of claim 10 to 13, wherein polymer particle is obtained using monomer mixture in which at least 10 weight % of all monomers are cross-linking monomers, wherein particles have pore structure.

**15.** The manufacturing method of a polymer particle of any of Claims 10 to 14, wherein vinyl alcohol unit is formed through hydrolysis and/or ester exchange reaction of part or all of carboxylic vinyl ester units of cross-linked polymer that contains carboxylic vinyl ester unit obtained by polymerization of monomer mixture containing vinyl compound having carboxylic vinyl ester and polyfunctional vinyl compound.

**16.** The manufacturing method of a polymer particles of any of Claims 10 to 14, wherein particle surface is preferably modified by polymerization of monomer mixture containing at least one kind of monomer selected from the group consisting of carboxylic vinyl ester, methacrylate ester, acrylic ester, aromatic vinyl compound and vinyl cyanide compound, and simultaneously and/or subsequently, by graft polymerization of monomer that provides a hydrophilic group.

**17.** The manufacturing method of a polymer particle of any of claims 10 to 16, wherein uniformly-sized liquid drops are formed in dispersion medium by causing regular oscillation disturbance to liquid column of monomer mixture, spurted out into dispersion medium from a nozzle hole, and subsequently said liquid drops are polymerized under the condition that neither connation nor additional dispersion occurs.

**Patentansprüche**

**1.** Ein vernetztes Polymerteilchen, umfassend eine Vinylalkoholeinheit und eine Stickstoff enthaltende Polymerisationseinheit, worin der Stickstoffgehalt als Trockengewicht auf Basis des Gesamtgewichts der Teilchen, bestimmt durch Elementaranalyse, 7,0 bis 13,0 Gew.-% beträgt, und der Oberflächenstickstoffgehalt gegen die Teilchenoberfläche, bestimmt durch Röntgenphotoelektronenspektromkopie, 5,0 bis 15,0 at.-% beträgt, worin:

die Vinylalkoholeinheit gebildet wird durch Hydrolyse und/oder eine Esteraustauschreaktion eines Teils oder aller Carboxylvinylestereinheiten eines vernetzten Polymer, welches Carboxylvinylestereinheiten enthält, das erhalten wird durch Polymerisation einer Monomermischung, enthaltend einen Carboxylvinylester und eine Vinylverbindung mit einem Triazinring, und worin
die Polymerisationsumwandlungsrate der Carboxylvinylester in der Polymerisation der Monomermischung, enthaltend den Carboxylvinylester und die Vinylverbindung mit einem Triazenring, gleich 10 bis 80 % ist.

**2.** Das vernetzte Polymerteilchen nach Anspruch 1, worin der numerische Unterschied zwischen dem Prozentsatz an Stickstoffgehalt und dem Prozentsatz an Oberflächenstickstoffgehalt weniger als 2,0 ist

**3.** Das vernetzte Polymerteilchen nach Anspruch 1, umfassend ein synthetisches Polymer, enthaltend Vinylalkoholeinheiten und eine Stickstoff enthaltende Polymerisationseinheit, worin der Stickstoffgehalt als Trockengewicht, bestimmt durch Elementaranalyse, 7,3 bis 9,2 Gew.-% ist.

**4.** Das vernetzte Polymerteilchen nach Anspruch 1, umfassend eine Vinylalkoholeinheiten und eine Stickstoff enthaltende Polymerisationseinheit, worin der Prozentsatz an Stickstoff enthaltenden Polymerisationseinheiten gegen die gesamten Polymerisationseinheiten, welche das vernetzte Polymerteilchen aufbauen, gleich 41,0 bis 75,0 Gew.-% ist, worin der Prozentsatz an Stickstoff enthaltenden Polymerisationseinheiten auf der Oberfläche des vernetzten Polymerteilchens 30,0 bis 85,0 Gew.-% ist.

**5.** Das vernetzte Polymerteilchen nach Anspruch 4, worin der Unterschied zwischen dem Prozentsatz an Stickstoff enthaltenden Polymerisationseinheiten gegen die gesamten Polymerisationseinheiten, welche das vernetzte Polymerteilchen aufbauen, und der Prozentsatz an Stickstoff enthaltenden Polymerisationseinheiten auf der Oberfläche der Polymerteilchen weniger als 15 Gew.-% beträgt.

**6.** Das vernetzte Polymerteilchen nach Anspruch 1, umfassend ein synthetisches Polymer, umfassend Vinylalkoholeinheiten und Stickstoff enthaltende Polymerisationseinheiten, worin der Prozentsatz an Stickstoff enthaltenden Polymerisationseinheiten gegen die gesamten Polymerisationseinheiten, die das vernetzte Polymerteilchen aufbauen, gleich 43,0 bis 54,0 Gew.-% ist.

**7.** Das vernetzte Polymerteilchen nach einem der Ansprüche 1 bis 6, worin der volumengemittelte mittlere Durchmesser der vernetzten Polymerteilchen gleich 50 bis 3000 $\mu$m ist, worin mehr als 80 Vol.-% dieser Teilchen den 0,8- bis 1,2-fache volumengemittelten mittleren Durchmesser aufweisen.

**8.** Das vernetzte Polymerteilchen nach einem der Ansprüche 1 bis 7, worin Teilchen mit einer Teilchengröße von weniger als 100 $\mu$m 5 Vol.-% entsprechen.

**9.** Ein Verarbeitungsmaterial einer Körperflüssigkeit, umfassend das vernetzte Polymer nach einem der Ansprüche 1 bis 8.

**10.** Ein Verarbeitungsverfahren eines Polymerteilchens, umfassend ein Verfahren (a) um flüssige Tropfen einheitlicher Größe einer Monomermischung, umfassend Vinylalkoholeinheiten und Stickstoff enthaltende Polymerisationseinheiten, in einem Dispersionsmedium zu bilden und ein Verfahren (b) um die flüssigen Tropfen unter Bedingungen zu polymerisieren, dass weder eine Verwachsung noch eine weitere Dispersion auftritt, worin der volumengemittelte mittlere Durchmesser 50 bis 3000 $\mu$m beträgt und 80 Vol.-% der Teilchen den 0,8- bis 1,2-fache volumengemittelten mittleren Durchmesser aufweisen, worin der Stickstoffgehalt als Trockengewicht gegen das Gesamtgewicht der Teilchen, bestimmt durch Elementaranalyse, gleich 7,0 bis 13,0 Gew.-% beträgt, und der Oberflächenstickstoffgehalt gegen die Teilchenoberfläche, bestimmt durch Röntgenphotoelektronenspektrometrie, gleich 5,0 bis 15,0 at.-% beträgt, worin die Vinylalkoholeinheit durch Hydrolyse und/oder Esteraustauschreaktion eines Teils oder aller Carboxylvinylestereinheiten des vernetzten Polymer gebildet wird, das erhalten wird durch Polymerisation einer Monomermischung, enthaltend einen Carboxylvinylester und eine Vinylverbindung mit einem Triazinring, und worin die Polymerisationsumwandlungsrate des Carboxylvinylesters in der Polymerisation der Monomermischung, enthaltend den Carboxylvinylester und die Vinylverbindung mit einem Triazinring, gleich 10 bis 80 % ist.

**11.** Das Herstellungsverfahren eines Polymerteilchens nach Anspruch 10, umfassend ein Verfahren (c) zu Durchführung und Modifikation nach dem Verfahren (b).

**12.** Das Herstellungsverfahren eines Polymerteilchens nach Anspruch 10 oder 11, worin der volumengemittelte mittlere Durchmesser der Polymerteilchen 150 bis 3000 $\mu$m beträgt, worin Teilchen von weniger als 100 $\mu$m an Partikelgröße höchstens 5 Vol.-% ausmachen.

**13.** Das Herstellungsverfahren eines Polymerteilchens nach einem der Ansprüche 10 bis 12, worin das Polymerteilchen mindestens einer Art an hydrophiler Gruppe, ausgewählt aus Hydroxyl, Polyoxyethylen und Pyrrolidon, aufweist.

**14.** Das Herstellungsverfahren für ein Polymerteilchen nach einem der Ansprüche 10 bis 13, worin das Polymerteilchen erhalten wird unter Verwendung einer Monomermischung, in welcher mindestens 10 Gew.-% aller Monomere gleich vernetzte Monomere sind, worin die Teilchen eine Porenstruktur aufweisen.

**15.** Das Herstellungsverfahren für ein Polymerteilchen nach einem der Ansprüche 10 bis 14, worin die Vinylalkoholeinheit gebildet wird durch Hydrolyse und/oder Esteraustauschreaktion eines Teils oder aller Carboxylvinylestereinheiten des vernetzten Polymer, welches die Carboxylvinylestereinheiten enthält, erhalten durch Polymerisation einer Monomermischung, enthaltend eine Vinylverbindung mit Carboxylvinylestern und einer polyfunktionellen Vinylverbindung.

16. Das Herstellungsverfahren für ein Polymerteilchen nach einem der Ansprüche 10 bis 14, worin die Teilchenoberfläche vorzugsweise modifiziert ist durch Polymerisation einer Monomermischung, enthaltend mindestens eine Form eines Monomers, ausgewählt aus der Gruppe, bestehend aus Carboxylvinylester, Methacrylatester, Acrylester, aromatischer Vinylverbindung und Vinylcyanidverbindung, und gleichzeitige und/oder anschließender Pfropfpolymerisation des Monomers, wodurch eine hydrophile Gruppe gebildet wird.

17. Das Herstellungsverfahren für ein Polymerteilchen nach einem der Ansprüche 10 bis 16, worin die flüssigen Tropfen einheitlicher Größe gebildet werden in einem Dispersionsmedium durch Einwirkung einer regelmäßigen Schwinkungsstörung in einer Flüssigkeitskolonne einer Monomermischung, Eintragen in ein Dispersionsmedium aus einer Lochdüse und anschließend werden die flüssigen Tropfen unter den Bedingungen polymerisiert, dass weder eine Verwachsung noch eine weitere Dispersion auftritt.

## Revendications

1. Particule de polymère réticulée comprenant des unités d'alcool vinylique et des unités de polymérisation contenant de l'azote, dans laquelle la teneur en azote selon le poids à sec par rapport au poids total de ladite particule, déterminée par l'analyse élémentaire est 7,0 à 13,0 % en poids, et la teneur en azote de surface par rapport à ladite surface de particule, déterminée par spectrométrie photoélectronique X est 5,0 à 15,0 % atomique, dans laquelle ladite unité d'alcool vinylique est formée par hydrolyse et/ou réaction d'échange ester d'une partie ou toutes les unités d'ester vinylique du polymère réticulé qui contient des unités d'ester de vinyle carboxylique obtenu par polymérisation de mélange monomère contenant d'ester de vinyle carboxylique et de composé vinylique ayant l'anneau triazine, et dans laquelle
le taux de conversion de polymérisation d'ester de vinyle carboxylique lors de la polymérisation de mélange monomère contenant d'ester de vinyle carboxylique et de composé vinylique ayant l'anneau triazine est 10 à 80 %.

2. Particule de polymère réticulée selon la revendication 1, dans laquelle la différence numérique entre le pourcentage de ladite teneur en azote et le pourcentage de ladite teneur en azote de surface est moins de 2.0.

3. Particule de polymère réticulée selon la revendication 1, comprenant de polymère synthétique incluant des unités d'alcool vinylique et des unités de polymérisation contenant de l'azote, dans laquelle la teneur en azote selon le poids à sec déterminée par l'analyse élémentaire est 7,3 à 9,2 % en poids.

4. Particule de polymère réticulée selon la revendication 1, comprenant des unités d'alcool vinylique et des unités de polymérisation contenant de l'azote, dans laquelle le pourcentage d'unité de polymérisation contenant de l'azote par rapport aux unités de polymérisation totales qui constituent la particule de polymère réticulée est 41,0 à 75,0 % en poids, dans laquelle le pourcentage d'unité de polymérisation contenant de l'azote sur la surface desdites particules de polymère réticulées est 30,0 à 85,0 % en poids.

5. Particule de polymère réticulée selon la revendication 4, dans laquelle la différence entre le pourcentage d'unité de polymérisation contenant de l'azote par rapport aux unités de polymérisation totales qui constituent la particule de polymère réticulée et le pourcentage d'unité de polymérisation contenant de l'azote sur la surface desdites particules de polymère est moins de 15,0 % en poids.

6. Particule de polymère réticulée selon la revendication 1, comprenant de polymère synthétique incluant des unités d'alcool vinylique et des unités de polymérisation contenant de l'azote, dans laquelle le pourcentage d'unité de polymérisation contenant de l'azote par rapport aux unités de polymérisation totales qui constituent la particule de polymère réticulée est 43,0 à 54,0 % en poids.

7. Particule de polymère réticulée selon l'une des revendications 1 à 6, dans laquelle le diamètre moyen sur base volumique desdites particules de polymère réticulées est 50 à 3000 $\mu$m, dans laquelle plus de 80 % volume desdites particules ont le diamètre moyen sur base volumique qui est 0,8 à 1,2 fois plus grand.

8. Particule de polymère réticulée selon l'une des revendications 1 à 7, dans laquelle des particules de moins de 100 $\mu$m de taille de particule constituent 5 % volume.

9. Matériau de traitement pour fluide corporel comprenant le polymère réticulé selon l'une des revendications 1 à 8.

10. Méthode de fabrication d'une particule de polymère, comprenant un procédé (a) afin de former des gouttes de liquide de taille uniforme de mélange de monomère comprenant des unités d'alcool vinylique et des unités de polymérisation contenant de l'azote dans milieu de dispersion et un procédé (b) afin de polymériser lesdites gouttes de liquide à condition que ni agglomération ni dispersion additionnelle ont lieu, dans laquelle le diamètre moyen sur base volumique est 50 à 3000 $\mu$m, et 80 % volume desdites particules ont le diamètre moyen sur base volumique qui est 0,8 à 1,2 fois plus grand, dans laquelle la teneur en azote selon le poids à sec par rapport au poids total de ladite particule, déterminée par l'analyse élémentaire est 7,0 à 13,0 % en poids, et la teneur en azote de surface par rapport à ladite surface de particule, déterminée par spectrométrie photoélectronique X est 5,0 à 15,0 % atomique, dans laquelle ladite unité d'alcool vinylique est formée par hydrolyse et/ou réaction d'échange ester d'une partie ou toutes les unités d'ester vinylique du polymère réticulé qui contient des unités d'ester de vinyle carboxylique obtenu par polymérisation de mélange monomère contenant d'ester de vinyle carboxylique et de composé vinylique ayant l'anneau triazine, et dans laquelle le taux de conversion de polymérisation d'ester de vinyle carboxylique lors de la polymérisation de mélange monomère contenant d'ester de vinyle carboxylique lors de la polymérisation de mélange de monomère contenant d'ester de vinyle carboxylique et de composé vinylique ayant l'anneau triazine est 10 à 80%.

11. Méthode de fabrication selon la revendication 10, comprenant le procédé (c) de traitement et de modification après ledit procédé (b).

12. Méthode de fabrication d'une particule de polymère selon les revendications 10 ou 11, dans laquelle le diamètre moyen sur base volumique des particules de polymère est 150 à 3000 $\mu$m, dans laquelle des particules de moins de 100 $\mu$m de taille de particule constituent 5 % volume au maximum.

13. Méthode de fabrication d'une particule de polymère selon les revendications 10 à 12, dans laquelle la particule de polymère a au moins une sorte de groupe hydrophile, sélectionnée parmi l'hydroxyle, le polyoxyéthylène et la pyrrolidone.

14. Méthode de fabrication d'une particule de polymère selon les revendications 10 à 13, dans laquelle la particule de polymère est obtenue en utilisant du mélange de monomère dans lequel au moins 10 % en poids de tous les monomères sont des monomères de réticulation, dans lequel les particules ont une structure de pores.

15. Méthode de fabrication d'une particule de polymère selon les revendications 10 à 14, dans laquelle l'unité d'alcool vinylique est formée par hydrolyse et/ou réaction d'échange ester d'une partie ou toutes les unités d'ester de vinyle carboxylique de polymère réticulé qui contient des unités d'ester de vinyle carboxylique obtenu par polymérisation de mélange monomère contenant de composé vinylique ayant d'ester de vinyle carboxylique et de composé vinylique polyfonctionnel.

16. Méthode de fabrication d'une particule de polymère selon les revendications 10 à 14, dans laquelle la surface de particule est préférablement modifiée par polymérisation de mélange monomère contenant au moins une sorte de monomère sélectionnée parmi le groupe consistant en l'ester de vinyle carboxylique, l'ester de méthacrylate, le composé aromatique de vinyle et le composé de cyanure de vinyle, et simultanément et/ou par la suite par polymérisation par greffe de monomère qui fournit un groupe hydrophile.

17. Méthode de fabrication d'une particule de polymère selon les revendications 10 à 16, dans laquelle des gouttes de liquide de taille uniforme sont formées dans un milieu de dispersion par le fait de causer une dérangement de l'oscillation régulière de colonne de liquide de mélange monomère, giclé dans un milieu de dispersion à partir d'un trou de gicleur et ensuite lesdites gouttes de liquide sont polymérisées sur la condition que ni agglomération ni dispersion additionnelle apparaissent.

**EP 1 832 607 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 58012656 A
- JP 63115572 A
- JP 2199137 A
- JP 60090038 A
- EP 0043074 A

- JP 56151703 A
- JP 60179404 A
- JP 57190003 A
- JP 58109504 A

**Non-patent literature cited in the description**

- **ICHIKAWA et al.** *Journal of Artificial Organs,* 1983, vol. 12 (1), 116-119